# EUROPEAN PATENT SPECIFICATION

(11) **EP 4 472 635 B1**
(45) Date of publication and mention of the grant of the patent: **22.04.2026**
(21) Application number: 23709805.8
(22) Date of filing: 02.02.2023
(51) Int. Cl.: A61K 31/47, A61K 31/404, A61K 31/437, A61K 31/4375, A61K 31/4439, A61K 31/444, A61K 31/519, A61K 45/06, A61P 11/00

(54) **METHODS OF TREATMENT FOR CYSTIC FIBROSIS**
VERFAHREN ZUR BEHANDLUNG VON ZYSTISCHER FIBROSE
MÉTHODES DE TRAITEMENT DE LA FIBROSE KYSTIQUE

(30) Priority: 03.02.2022 US 202263306344 P
(43) Date of publication of application: 11.12.2024
(73) Proprietor: Vertex Pharmaceuticals Incorporated, Boston, MA 02210 (US)
(72) Inventor: BOREK, Bartlomiej, Boston, Massachusetts 02210 (US); LATERREUR, Julie, Boston, Massachusetts 02210 (US); LU, Jennifer, Boston, Massachusetts 02210 (US); PRIETO CENTURION, Valentin, Boston, MA 02210 (US); RHEE, David, Boston, MA 02210 (US)
(74) Representative: Carpmaels & Ransford LLP
(86) International application number: PCT/US2023/012230
(87) International publication number: WO 2023/150237

(56) References cited:
- WO-A1-2013/038386
- WO-A1-2019/018395
- WO-A1-2019/161078
- WO-A1-2022/032068
- WO-A1-2022/109573
- US-B2- 10 570 115

## Description

This application claims the benefit of U.S. Provisional Application No. 63/306,344, filed on February 3, 2022.

The invention relates to modulators of Cystic Fibrosis Transmembrane Conductance Regulator (CFTR), pharmaceutical compositions containing the modulators, methods of treatment of cystic fibrosis and CFTR-mediated disorders using such modulators and pharmaceutical compositions, and processes for making such modulators.

Cystic fibrosis (CF) is a recessive genetic disease that affects approximately 88,000 children and adults worldwide. Despite progress in the treatment of CF, there is no cure.

In patients with CF, mutations in CFTR endogenously expressed in respiratory epithelia lead to reduced apical anion secretion causing an imbalance in ion and fluid transport. The resulting decrease in anion transport contributes to increased mucus accumulation in the lung and accompanying microbial infections that ultimately cause death in CF patients. In addition to respiratory disease, CF patients typically suffer from gastrointestinal problems and pancreatic insufficiency that, if left untreated, result in death. In addition, the majority of males with cystic fibrosis are infertile, and fertility is reduced among females with cystic fibrosis.

Sequence analysis of the CFTR gene has revealed a variety of disease-causing mutations (Cutting, G. R. et al. (1990) Nature 346:366-369; Dean, M. et al. (1990) Cell 61:863:870; and Kerem, B-S. et al. (1989) Science 245:1073-1080; Kerem, B-S. et al. (1990) Proc. Natl. Acad. Sci. USA 87:8447-8451). To date, greater than 2000 mutations in the CF gene have been identified; currently, the CFTR2 database contains information on at least 322 of these identified mutations, with sufficient evidence to define at least 281 mutations as disease-causing. The most prevalent disease-causing mutation is a deletion of phenylalanine at position 508 of the CFTR amino acid sequence and is commonly referred to as the F508del mutation. This mutation occurs in many of the cases of cystic fibrosis and is associated with severe disease.

CFTR is a cAMP/ATP-mediated anion channel that is expressed in a variety of cell types, including absorptive and secretory epithelia cells, where it regulates anion flux across the membrane, as well as the activity of other ion channels and proteins. In epithelial cells, normal functioning of CFTR is critical for the maintenance of electrolyte transport throughout the body, including respiratory and digestive tissue. CFTR is composed of 1480 amino acids that encode a protein which is made up of a tandem repeat of transmembrane domains, each containing six transmembrane helices and a nucleotide binding domain. The two transmembrane domains are linked by a large, polar, regulatory (R)-domain with multiple phosphorylation sites that regulate channel activity and cellular trafficking.

Chloride transport takes place by the coordinated activity of ENaC (epithelial sodium channel) and CFTR present on the apical membrane and the Na⁺-K⁺-ATPase pump and Cl⁻ channels expressed on the basolateral surface of the cell. Secondary active transport of chloride from the luminal side leads to the accumulation of intracellular chloride, which can then passively leave the cell via Cl⁻ channels, resulting in a vectorial transport. Arrangement of Na⁺/2Cl⁻/K⁺ co-transporter, Na⁺-K⁺-ATPase pump and the basolateral membrane K⁺ channels on the basolateral surface and CFTR on the luminal side coordinate the secretion of chloride. Because water is probably never actively transported itself, its flow across epithelia depends on tiny transepithelial osmotic gradients generated by the bulk flow of sodium and chloride.

A number of CFTR modulators have recently been identified. These modulators can be characterized as, for example, potentiators, correctors, potentiator enhancers/co-potentiators, amplifiers, readthrough agents, and nucleic acid therapies. CFTR modulators that increase the channel gating activity of mutant and wild-type CFTR at the epithelial cell surface are known as potentiators. Correctors improve faulty protein processing and resulting trafficking to the epithelial surface. Ghelani and Schneider-Futschik (2020) ACS Pharmacol. Transl. Sci. 3:4-10. There are three CFTR correctors approved by the U.S. FDA for treatment of cystic fibrosis. However, monotherapy with some CFTR correctors has not been found to be effective enough and as a result combination therapy with a potentiator is often needed to enhance CFTR activity. There is currently only one CFTR potentiator that is approved for the treatment of cystic fibrosis.
WO 2013/038386 relates to pyridine-oxadiazole/thiadiazole based compounds, and pharmaceutical compositions thereof, which are said to "restore or enhance the function of mutant and/or wild type CFTR to treat cystic fibrosis, primary ciliary dyskinesia, chronic bronchitis, chronic obstructive pulmonary disease, asthma, respiratory tract infections, lung carcinoma, xerostomia and keratoconjunctivitis sire, or constipation (IBS, IBD, opioid induced)".
WO 2019/018395 relates to a compound, and/or pharmaceutically acceptable salts thereof, comprised in a pharmaceutical composition and methods of using the same to treat cystic fibrosis.
Accordingly, there is a need for novel treatments of cystic fibrosis and other CFTR mediated diseases.
The present invention is defined by the claims. Any subject-matter falling outside the scope of the claims is provided for information only.
Methods of treatment of the human body by therapy, referred to in this description, are not part of the present invention as such, but are described here in relation to compounds and pharmaceutical compositions for use in said methods for treatment of the human body by therapy, according to the present invention.

Thus, one aspect of the disclosure provides a CFTR-modulating compound, (6R,12R)-17-amino-12-methyl-6,15-bis(trifluoromethyl)-13,19-dioxa-3,4,18-triazatricyclo[12.3.1.12,5]nonadeca-1(18),2,4,14,16-pentaen-6-ol (Compound **I),** deuterated derivatives thereof, and pharmaceutically acceptable salts of any of the foregoing. Compound **I** can be depicted as having the following structure:

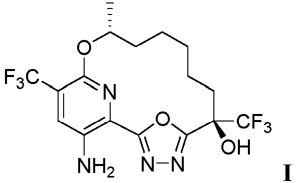

Other aspects of the disclosure provide pharmaceutical compositions comprising Compound **I,** a deuterated derivative thereof, or a pharmaceutically acceptable salt of any of the foregoing, which compositions may further include at least one additional active pharmaceutical ingredient and/or at least one pharmaceutically acceptable carrier. Yet other aspects of the disclosure are methods of treating the CFTR-mediated disease cystic fibrosis comprising administering Compound **I,** a deuterated derivative thereof, or a pharmaceutically acceptable salt of any of the foregoing, optionally as part of a pharmaceutical composition comprising at least one additional component, to a subject in need thereof.

Another aspect of the invention provides methods of treating the CFTR-mediated disease cystic fibrosis comprising administering to a patient in need thereof Compound **I,** a deuterated derivative thereof, or a pharmaceutically acceptable salt of any of the foregoing, alone or in combination with one or more additional CFTR-modulating agents selected from (*R*)-1-(2,2-difluorobenzo[d][1,3]dioxol-5-yl)-*N*-(1-(2,3-dihydroxypropyl)-6-fluoro-2-(1-hydroxy-2-methylpropan-2-yl)-1*H*-indol-5-yl)cyclopropanecarboxamide (Compound **II**), *N*-[2,4-bis(1,1-dimethylethyl)-5-hydroxyphenyl]-1,4-dihydro-4-oxoquinoline-3-carboxamide (also known as *N*-[2,4-bis(1,1-dimethylethyl)-5-hydroxyphenyl]-1,4-dihydro-4-oxoquinoline-3-carboxamide) (Compound **III)** or *N*-(2-(tert-butyl)-5-hydroxy-4-(2-(methyl-d3)propan-2-yl-1,1,1,3,3,3-d6)phenyl)-4-oxo-1,4-dihydroquinoline-3-carboxamide (Compound **III-d),** 3-(6-(1-(2,2-difluorobenzo[d][1,3]dioxol-5-yl)cyclopropane carboxamido)-3-methylpyridin-2-yl)benzoic acid (Compound **IV),** *N*-(1,3-dimethylpyrazol-4-yl)sulfonyl-6-[3-(3,3,3-trifluoro-2,2-dimethyl-propoxy)pyrazol-1-yl]-2-[(4*S*)-2,2,4-trimethylpyrrolidin-1-yl]pyridine-3-carboxamide (Compound **V),** *N*-(benzenesulfonyl)-6-[3-[2-[1-(trifluoromethyl) cyclopropyl]ethoxy]pyrazol-1-yl]-2-[(4*S*)-2,2,4-trimethylpyrrolidin-l-yl]pyridine-3-carboxamide (Compound **VI),** (14*S*)-8-[3-(2-{dispiro[2.0.2.1]heptan-7-yl}ethoxy)-1*H*-pyrazol-1-yl]-12,12-dimethyl-2λ⁶-thia-3,9,11,18,23-pentaazatetracyclo [17.3.1.111,14.05,10]tetracosa-1(22),5,7,9,19(23),20-hexaene-2,2,4-trione (Compound **VII),** (11*R*)-6-(2,6-dimethylphenyl)-11-(2-methylpropyl)-12-{spiro[2.3]hexan-5-yl}-9-oxa-2λ⁶-thia-3,5,12,19-tetraazatricyclo[12.3.1.14,8]nonadeca-1(17),4(19),5,7,14(18),15-hexaene-2,2,13-trione (Compound **VIII);** *N*-(benzenesulfonyl)-6-(3-fluoro-5-isobutoxy-phenyl)-2-[(4*S*)-2,2,4-trimethylpyrrolidin-l-yl]pyridine-3-carboxamide (Compound **IX),** and *N*-[(6-amino-2-pyridyl)sulfonyl]-6-(3-fluoro-5-isobutoxy-phenyl)-2-[(4*S*)-2,2,4-trimethylpyrrolidin-1-yl]pyridine-3-carboxamide (Compound **X).** In some embodiments, Compound **I,** a deuterated derivative thereof, or a pharmaceutically acceptable salt of any of the foregoing, is administered in the same composition with at least one compound chosen from Compound **II,** Compound **III,** Compound **III-d,** Compound **IV,** Compound **V,** Compound **VI,** Compound **VII,** Compound **VIII,** Compound **IX,** Compound **X,** deuterated derivatives thereof, and pharmaceutically acceptable salts of any of the foregoing. In some embodiments, a composition comprising Compound **I**, a deuterated derivative thereof, or a pharmaceutically acceptable salt of any of the foregoing, is co-administered with a separate composition comprising at least one compound chosen from Compound **II,** Compound **III,** Compound **III-d,** Compound **IV,** Compound **V,** Compound **VI,** Compound **VII,** Compound **VIII,** Compound **IX,** Compound **X,** deuterated derivatives thereof, and pharmaceutically acceptable salts of any of the foregoing.

Another aspect of the invention provides methods of treating the CFTR-mediated disease cystic fibrosis comprising administering to a patient in need thereof Compound **I,** a deuterated derivative thereof, or a pharmaceutically acceptable salt of any of the foregoing, and optionally further administering one or more additional CFTR-modulating agents selected from ASP-11, disclosed in Journal of Cystic Fibrosis (2018), 17(5), 595-606, and nesolicaftor or PTI-428, disclosed in WO 2016/105485. In one embodiment, the additional CFTR-modulating agent is ASP-11. In one embodiment, the additional CFTR-modulating agent comprises PTI-428.

Another aspect of the invention provides methods of treating the CFTR-mediated disease cystic fibrosis comprising administering to a patient in need thereof Compound **I,** a deuterated derivative thereof, or a pharmaceutically acceptable salt of any of the foregoing, and optionally further administering one or more additional CFTR-modulating agents selected from galicaftor or ABBV-2222, disclosed in United States Patent Application Publication No. 2016-0120841; ABBV-3221, disclosed in WO 2018/065921; posenacaftor or PTI-801, disclosed in WO 2017/062581; ABBV-2851, disclosed in WO 2017/009804; GLPG2737, disclosed in United States Patent Application Publication No. 2017-0101405; ABBV-3748; ABBV-3903; and ABBV-119.

In certain embodiments, the pharmaceutical compositions of the invention comprise Compound **I,** a deuterated derivative thereof, or a pharmaceutically acceptable salt of any of the foregoing, and may optionally further comprise at least one compound chosen from Compound **II,** Compound **III,** Compound **III-d,** Compound **IV,** Compound **V,** Compound **VI,** Compound **VII,** Compound **VIII,** Compound **IX,** Compound **X,** deuterated derivatives thereof, and pharmaceutically acceptable salts of any of the foregoing.

### Brief Description of the Figures

**FIG. 1** provides a Study Design for Part A of a first-in-human (FIH) study of Compound **I** and provides the doses that were studied.
**FIG. 2** provides a Study Design for Part B of a FIH study of Compound **I** and provides the doses that were studied.
**FIG. 3** provides a Study Design for Part C of a FIH study of Compound **I**.

### Definitions

"Compound **I"** as used throughout this disclosure refers to (6*R*,12*R*)-17-amino-12-methyl-6,15-bis(trifluoromethyl)-13,19-dioxa-3,4,18-triazatricyclo[12.3.1.12,5]nonadeca-1(18),2,4,14,16-pentaen-6-ol, which can be depicted as having the following structure:

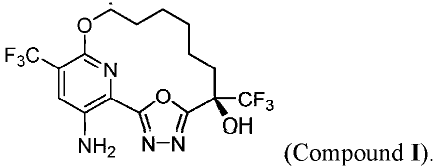

Compound **I** may be a racemic mixture or an enantioenriched (e.g., >90% ee, >95% ee, > 98% ee) mixture of isomers. Compound **I** or a deuterated derivative thereof may be in the form of a pharmaceutically acceptable salt, solvate, and/or hydrate. Compound **I** and methods for making and using Compound **I** are disclosed in PCT International Application No. PCT/US2021/044895, which published as WO 2022/032068.

"Compound **II,"** as used herein, refers to (*R*)-1-(2,2-difluorobenzo[d][1,3]dioxol-5-yl)-*N*(1-(2,3-dihydroxypropyl)-6-fluoro-2-(1-hydroxy-2-methylpropan-2-yl)-1*H*-indol-5-yl)cyclopropanecarboxamide, which can be depicted with the following structure: 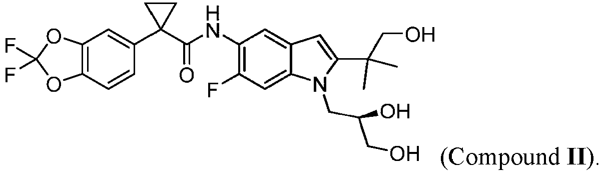 Compound **II** may be in the form of a pharmaceutically acceptable salt. Compound **II** and methods of making and using Compound **II** are disclosed in WO 2010/053471, WO 2011/119984, WO 2011/133751, WO 2011/133951, and WO 2015/160787.

"Compound **III"** as used throughout this disclosure refers to *N*-(5-hydroxy-2,4-di-*tert*-butyl-phenyl)-4-oxo-1H-quinoline-3-carboxamide (also known as *N*-[2,4-bis(1,1-dimethylethyl)-5-hydroxyphenyl]-1,4-dihydro-4-oxoquinoline-3-carboxamide), which is depicted by the structure: 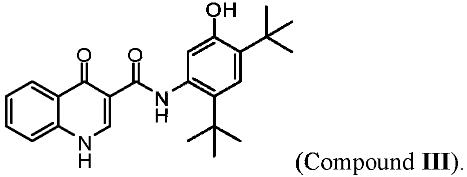 Compound **III** may also be in the form of a pharmaceutically acceptable salt. Compound **III** and methods of making and using Compound **III** are disclosed in WO 2006/002421, WO 2007/079139, WO 2010/108162, and WO 2010/019239.

In some embodiments, a deuterated derivative of Compound **III** (Compound **III-d)** is employed in the compositions and methods disclosed herein. A chemical name for Compound **III-d** is *N*-(2-(*tert*-butyl)-5-hydroxy-4-(2-(methyl-d3)propan-2-yl-1,1,1,3,3,3-d6)phenyl)-4-oxo-1,4-dihydroquinoline-3-carboxamide, as depicted by the structure: 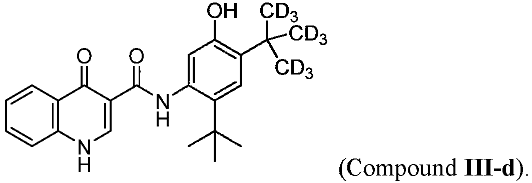 Compound **III-d** may be in the form of a pharmaceutically acceptable salt. Compound **III-d** and methods of making and using Compound **III-d** are disclosed in WO 2012/158885, WO 2014/078842, WO 2019/109021, and US Patent No. 8,865,902.

"Compound **IV"** as used herein, refers to 3-(6-(1-(2,2-difluorobenzo[d][1,3]dioxol-5-yl)cyclopropanecarboxamido)-3-methylpyridin-2-yl)benzoic acid, which is depicted by the chemical structure: 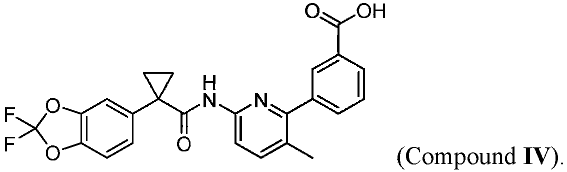 Compound **IV** may be in the form of a pharmaceutically acceptable salt. Compound **IV** and methods of making and using Compound **IV** are disclosed in WO 2007/056341, WO 2009/073757, and WO 2009/076142.

"Compound **V"** as used herein, refers to *N*-(1,3-dimethylpyrazol-4-yl)sulfonyl-6-[3-(3,3,3-trifluoro-2,2-dimethyl-propoxy)pyrazol-1-yl]-2-[(4S)-2,2,4-trimethylpyrrolidin-1-yl]pyridine-3-carboxamide, which is depicted by the chemical structure: 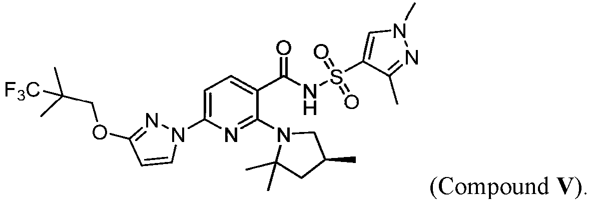 Compound **V** may be in the form of a pharmaceutically acceptable salt. Compound **V** and methods of making and using Compound V are disclosed in WO 2018/107100 and WO 2019/113476.

"Compound **VI"** as used herein, refers to *N*-(benzenesulfonyl)-6-[3-[2-[1-(trifluoromethyl) cyclopropyl]ethoxy]pyrazol-1-yl]-2-[(4*S*)-2,2,4-trimethylpyrrolidin-1-yl]pyridine-3-carboxamide, which is depicted by the chemical structure: 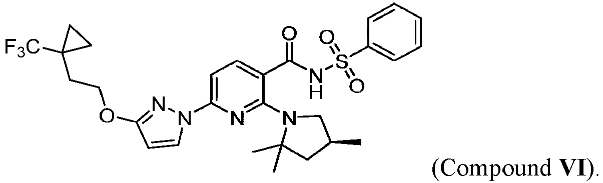 Compound **VI** may be in the form of a pharmaceutically acceptable salt. Compound **VI** and methods of making and using Compound **VI** are disclosed in WO 2018/064632 and WO 2019/113476.

"Compound **VII**" as used herein, refers to (14*S*)-8-[3-(2-{dispiro[2.0.2.1]heptan-7-yl}ethoxy)-1*H*-pyrazol-l-yl]-12,12-dimethyl-2λ⁶-thia-3,9,11,18,23-pentaazatetracyclo [17.3.1.111,14.05,10]tetracosa-1(22),5,7,9,19(23),20-hexaene-2,2,4-trione, which is depicted by the chemical structure: 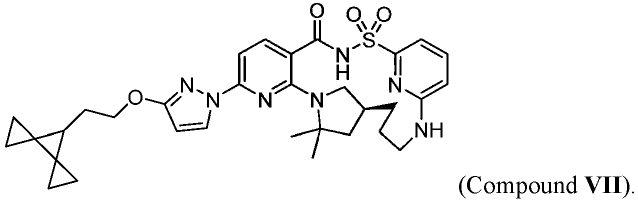 Compound **VII** may be in the form of a pharmaceutically acceptable salt. Compound **VII** and methods of making and using Compound **VII** are disclosed in WO 2019/161078, WO 2020/102346, and WO 2021/030554.

"Compound **VIII"** as used herein, refers to (11*R*)-6-(2,6-dimethylphenyl)-11-(2-methylpropyl)-12-{spiro[2.3]hexan-5-yl}-9-oxa-2λ⁶-thia-3,5,12,19-tetraazatricyclo[12.3.1.14,8]nonadeca-1(17),4(19),5,7,14(18),15-hexaene-2,2,13-trione, which is depicted by the chemical structure: 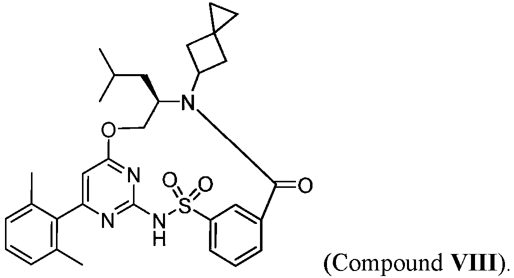 Compound **VIII** may be in the form of a pharmaceutically acceptable salt. Compound **VIII** and methods of making and using Compound **VIII** are disclosed in WO 2020/206080.

"Compound **IX"** as used herein, refers to *N*-(benzenesulfonyl)-6-(3-fluoro-5-isobutoxy-phenyl)-2-[(4*S*)-2,2,4-trimethylpyrrolidin-1-yl]pyridine-3-carboxamide, which is depicted by the chemical structure: 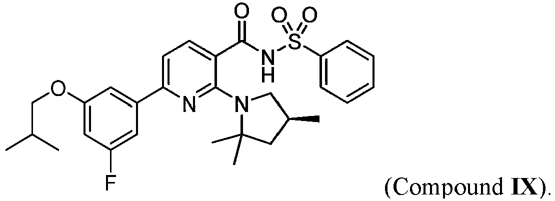 Compound **IX** may be in the form of a pharmaceutically acceptable salt. Compound **IX** and methods of making and using Compound **IX** are disclosed in WO 2016/057572.

"Compound **X"** as used herein, refers to *N*-[(6-amino-2-pyridyl)sulfonyl]-6-(3-fluoro-5-isobutoxy-phenyl)-2-[(4*S*)-2,2,4-trimethylpyrrolidin-1-yl]pyridine-3-carboxamide, which is depicted by the chemical structure: 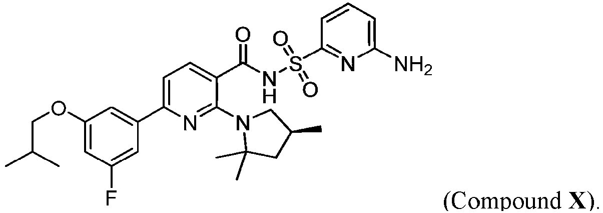 Compound **X** may be in the form of a pharmaceutically acceptable salt. Compound **X** and methods of making and using Compound **X** are disclosed in WO 2016/057572.

As used herein, "CFTR" means cystic fibrosis transmembrane conductance regulator.

As used herein, "mutations" can refer to mutations in the *CFTR* gene or the CFTR protein. A *"CFTR* gene mutation" refers to a mutation in the *CFTR* gene, and a "CFTR protein mutation" refers to a mutation in the CFTR protein. A genetic defect or mutation (i.e., a change in one or more nucleotides) in a gene in general results in a mutation (i.e., a change in one or more amino acids) in the CFTR protein translated from that gene, or a frame shift (i.e., a change in the reading frame that governs how the *CFTR* gene is translated into the CFTR protein).

As used herein, the term "F508del" refers to a mutant CFTR protein which is lacking the amino acid phenylalanine at position 508, or to a mutant *CFTR* gene which encodes for a CFTR protein lacking the amino acid phenylalanine at position 508.

As used herein, a patient who is "homozygous" for a particular gene mutation has the same mutation on each allele.

As used herein, a patient who is "heterozygous" for a particular gene mutation has this mutation on one allele, and a different mutation on the other allele.

As used herein, the terms "CFTR modulator," "CFTR-modulating compound," and "CFTR-modulating agent" interchangeably refer to a compound that directly or indirectly increases the activity of CFTR. CFTR modulators include but are not limited to compounds that correct, potentiate, stabilize, and/or amplify CFTR.

As used herein, the term "CFTR corrector" refers to a compound that facilitates the processing and trafficking of CFTR to increase the amount of CFTR at the cell surface. Compound **II,** Compound **IV,** Compound **V,** Compound **VI,** Compound **VII,** Compound **VIII,** Compound **IX,** and Compound **X** disclosed herein are CFTR correctors.

As used herein, the term "CFTR potentiator" refers to a compound that increases the channel activity of CFTR protein located at the cell surface, resulting in enhanced ion transport. Compound **III** and Compound **III-d** disclosed herein are CFTR potentiators.

As used herein, the term "active pharmaceutical ingredient" ("API") or "therapeutic agent" refers to a biologically active compound.

It will be appreciated that certain compounds of this invention may exist as separate stereoisomers or enantiomers and/or mixtures of those stereoisomers or enantiomers. As used in the chemical structures disclosed herein, a "wedge" ( 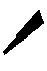 ) or "hash" ( 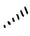 ) bond to a stereogenic atom indicates a chiral center of known absolute stereochemistry (i.e., one stereoisomer). As used in the chemical structures disclosed herein, a "wavy" bond ( 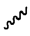 ) to a stereogenic atom indicates a chiral center of unknown absolute stereochemistry (i.e., one stereoisomer). As used in the chemical structures disclosed herein, a "wavy" bond ( 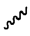 ) to a double-bonded carbon indicates a mixture of E/Z isomers. As used in the chemical structures disclosed herein, a 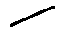 ("straight") bond to a stereogenic atom indicates where there is a mixture (e.g., a racemate or enrichment). As used herein, two 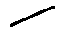 ("straight") bonds to a double-bonded carbon indicates that the double bond possesses the *E*/*Z* stereochemistry as drawn. As used in the chemical structures disclosed herein, a 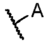 (i.e., a "wavy" line perpendicular to a "straight" bond to group "A") indicates that group "A" is a substituent whose point of attachment is at the end of the bond that terminates at the "wavy" line.

The terms "patient" and "subject" are used interchangeably and refer to an animal including humans.

The terms "effective dose" and "effective amount" are used interchangeably herein and refer to that amount of a compound that produces the desired effect for which it is administered (e.g., improvement in CF or a symptom of CF, or lessening the severity of CF or a symptom of CF). The exact amount of an effective dose will depend on the purpose of the treatment and will be ascertainable by one skilled in the art using known techniques (see, e.g., Lloyd (1999) The Art, Science and Technology of Pharmaceutical Compounding).

As used herein, the terms "treatment," "treating," and the like generally mean the improvement of CF or one or more of its symptoms or lessening the severity of CF or one or more of its symptoms in a subject. "Treatment," as used herein, includes, but is not limited to, the following: increased growth of the subject, increased weight gain, reduction of mucus in the lungs, improved pancreatic and/or liver function, reduction of chest infections, and/or reductions in coughing or shortness of breath. Improvements in or lessening the severity of any of these symptoms can be readily assessed according to standard methods and techniques known in the art.

As used herein, the term "in combination with," when referring to two or more compounds, agents, or additional APIs, means the administration of two or more compounds, agents, or APIs to the patient prior to, concurrently with, or subsequent to each other.

The terms "about" and "approximately," when used in connection with doses, amounts, or weight percentages of ingredients of a composition or a dosage form, include the value of a specified dose, amount, or weight percentage or a range of the dose, amount, or weight percentage that is recognized by one of ordinary skill in the art to provide a pharmacological effect equivalent to that obtained from the specified dose, amount, or weight percent. The terms "about" and "approximately" may refer to an acceptable error for a particular value as determined by one of skill in the art, which depends in part on how the values is measured or determined. In some embodiments, the terms "about" and "approximately" mean within 15%, 10%, 5%, 4%, 3%, 2%, 1%, or 0.5% of a given value or range. In some embodiments, the terms "about" and "approximately" mean within 15% of a given value. In some embodiments, the terms "about" and "approximately" mean within 10% of a given value.

As used herein, "minimal function (MF) mutations" refer to CFTR gene mutations associated with minimal CFTR function (little-to-no functioning CFTR protein) and include, for example, mutations associated with severe defects in ability of the CFTR channel to open and close, known as defective channel gating or "gating mutations"; mutations associated with severe defects in the cellular processing of CFTR and its delivery to the cell surface; mutations associated with no (or minimal) CFTR synthesis; and mutations associated with severe defects in channel conductance.

As used herein, "deuterated derivative(s)" means the same chemical structure, with one or more hydrogen atoms replaced by a deuterium atom.

As used herein, the term "pharmaceutically acceptable salt" refers to a salt form of a compound of this disclosure, or a deuterated derivative thereof, wherein the salt is nontoxic. Pharmaceutically acceptable salts of the compounds of this disclosure include those derived from suitable inorganic and organic acids and bases. A "free base" form of a compound, for example, does not contain an ionically bonded salt.

The phrase "deuterated derivative or pharmaceutically acceptable salt thereof" is used interchangeably with "deuterated derivative thereof or pharmaceutically acceptable salt of any of the forgoing" in reference to one or more compounds or formulae of the invention. The phrase "pharmaceutically acceptable salt and deuterated derivative thereof" is used interchangeably with "pharmaceutically acceptable salt thereof and deuterated derivative of any of the forgoing" in reference to one or more compounds or formulae of the invention. These phrases are intended to encompass pharmaceutically acceptable salts of any one of the referenced compounds, deuterated derivatives of any one of the referenced compounds, deuterated derivatives of pharmaceutically acceptable salts of any one of the referenced compounds, and pharmaceutically acceptable salts of deuterated derivatives of any one of the referenced compounds.

One of ordinary skill in the art would recognize that, when an amount of "a compound or a pharmaceutically acceptable salt thereof" is disclosed, the amount of the pharmaceutically acceptable salt form of the compound is the amount equivalent to the concentration of the free base of the compound. It is noted that the disclosed amounts of the compounds or their pharmaceutically acceptable salts thereof herein are based upon their free base form. For example, "100 mg of Compound I or a pharmaceutically acceptable salt thereof" or "100 mg of Compound I or an equivalent amount of a pharmaceutically acceptable salt thereof" includes 100 mg of Compound I and an amount of a pharmaceutically acceptable salt of Compound I equivalent to 100 mg of Compound **I**.

Suitable pharmaceutically acceptable salts are, for example, those disclosed in S. M. Berge, et al. J. Pharmaceutical Sciences, 1977, 66, 1-19. For example, **Table 1** of that article provides the following pharmaceutically acceptable salts:

**Table 1:**

| | | |
|---|---|---|
| Acetate | Iodide | Benzathine |
| Benzenesulfonate | Isethionate | Chloroprocaine |
| Benzoate | Lactate | Choline |
| Bicarbonate | Lactobionate | Diethanolamine |
| Bitartrate | Malate | Ethylenediamine |
| Bromide | Maleate | Meglumine |
| Calcium edetate | Mandelate | Procaine |
| Camsylate | Mesylate | Aluminum |
| Carbonate | Methylbromide | Calcium |
| Chloride | Methylnitrate | Lithium |
| Citrate | Methyl sulfate | Magnesium |
| Dihydrochloride | Mucate | Potassium |
| Edetate | Napsylate | Sodium |
| Edisylate | Nitrate | Zinc |
| Estolate | Pamoate (Embonate) | |
| Esylate | Pantothenate | |
| Fumarate | Phosphate/diphosphate | |
| Gluceptate | Polygalacturonate | |
| Gluconate | Salicylate | |
| Glutamate | Stearate | |
| Glycollylarsanilate | Subacetate | |
| Hexylresorcinate | Succinate | |
| Hydrabamine | Sulfate | |
| Hydrobromide | Tannate | |
| Hydrochloride | Tartrate | |
| Hydroxynaphthoate | Teociate | |
| | Triethiodide | |

Non-limiting examples of pharmaceutically acceptable acid addition salts include: salts formed with inorganic acids, such as hydrochloric acid, hydrobromic acid, phosphoric acid, sulfuric acid, or perchloric acid; salts formed with organic acids, such as acetic acid, oxalic acid, maleic acid, tartaric acid, citric acid, succinic acid or malonic acid; and salts formed by using other methods used in the art, such as ion exchange. Non-limiting examples of pharmaceutically acceptable salts include adipate, alginate, ascorbate, aspartate, benzenesulfonate, benzoate, bisulfate, borate, butyrate, camphorate, camphorsulfonate, citrate, cyclopentanepropionate, digluconate, dodecylsulfate, ethanesulfonate, formate, fumarate, glucoheptonate, glycerophosphate, gluconate, hemisulfate, heptanoate, hexanoate, hydroiodide, 2-hydroxy-ethanesulfonate, lactobionate, lactate, laurate, lauryl sulfate, malate, maleate, malonate, methanesulfonate, 2-naphthalenesulfonate, nicotinate, nitrate, oleate, oxalate, palmitate, pamoate, pectinate, persulfate, 3-phenylpropionate, phosphate, picrate, pivalate, propionate, stearate, succinate, sulfate, tartrate, thiocyanate, p-toluenesulfonate, undecanoate, and valerate salts. Pharmaceutically acceptable salts derived from appropriate bases include alkali metal, alkaline earth metal, ammonium, and N⁺(C₁₋₄alkyl)₄ salts. This disclosure also envisions the quaternization of any basic nitrogen-containing groups of the compounds disclosed herein. Suitable non-limiting examples of alkali and alkaline earth metal salts include sodium, lithium, potassium, calcium, and magnesium. Further non-limiting examples of pharmaceutically acceptable salts include ammonium, quaternary ammonium, and amine cations formed using counterions such as halide, hydroxide, carboxylate, sulfate, phosphate, nitrate, lower alkyl sulfonate, and aryl sulfonate. Other suitable, non-limiting examples of pharmaceutically acceptable salts include besylate and glucosamine salts.

### Detailed Description of Embodiments

### Methods of Treatment

Compound I, a deuterated derivative thereof, or a pharmaceutically acceptable salt of any of the foregoing, can act as a CFTR modulator, i.e., it modulates CFTR activity in the body. Individuals suffering from a mutation in the gene encoding CFTR may benefit from receiving a CFTR modulator. A CFTR mutation may affect CFTR quantity, i.e., the number of CFTR channels at the cell surface, or it may impact CFTR function, i.e., the functional ability of each channel to open and transport ions. Mutations affecting CFTR quantity include mutations that cause defective synthesis (Class I defect), mutations that cause defective processing and trafficking (Class II defect), mutations that cause reduced synthesis of CFTR (Class V defect), and mutations that reduce the surface stability of CFTR (Class VI defect). Mutations that affect CFTR function include mutations that cause defective gating (Class III defect) and mutations that cause defective conductance (Class IV defect). Some CFTR mutations exhibit characteristics of multiple classes. Certain mutations in the CFTR gene result in CF.

Thus, in some embodiments, the invention provides methods of treating, lessening the severity of, or symptomatically treating cystic fibrosis in a patient comprising administering to the patient an effective amount of Compound I, a deuterated derivative thereof, or a pharmaceutically acceptable salt of any of the foregoing, alone or in combination with another active ingredient, such as another CFTR-modulating agent. In some embodiments, the patient has an F508del/minimal function (MF) genotype, F508del/F508del genotype (homozygous for the F508del mutation), F508del/gating genotype, or F508del/residual function (RF) genotype. In some embodiments, the patient is heterozygous and has one F508del mutation. In some embodiments, the patient is homozygous for the N1303K mutation.

In some embodiments, 5 mg to 100 mg (e.g., 5 mg, 10 mg, 15 mg, 20 mg, 25 mg, 30 mg, 35 mg, 40 mg, 45 mg, 50 mg, 55 mg, 60 mg, 65 mg, 70 mg, 75 mg, 80 mg, 85 mg, 90 mg, 95 mg, or 100 mg) of Compound **I**, a deuterated derivative thereof, or a pharmaceutically acceptable salt of any of the foregoing, is administered daily. In some embodiments, 7 mg to 90 mg of Compound **I,** a deuterated derivative thereof, or a pharmaceutically acceptable salt of any of the foregoing, is administered daily. In some embodiments, 10 mg to 80 mg of Compound **I,** a deuterated derivative thereof, or a pharmaceutically acceptable salt of any of the foregoing, is administered daily. In some embodiments, 10 mg to 40 mg of Compound **I,** a deuterated derivative thereof, or a pharmaceutically acceptable salt of any of the foregoing, is administered daily. In some embodiments, 30 mg to 60 mg of Compound **I,** a deuterated derivative thereof, or a pharmaceutically acceptable salt of any of the foregoing, is administered daily. In some embodiments, 50 mg to 80 mg of Compound **I,** a deuterated derivative thereof, or a pharmaceutically acceptable salt of any of the foregoing, is administered daily.

In some embodiments, about 5 mg to about 100 mg (e.g., about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg) of Compound **I,** a deuterated derivative thereof, or a pharmaceutically acceptable salt of any of the foregoing, is administered daily. In some embodiments, about 7 mg to about 90 mg of Compound **I,** a deuterated derivative thereof, or a pharmaceutically acceptable salt of any of the foregoing, is administered daily. In some embodiments, about 10 mg to about 80 mg of Compound **I,** a deuterated derivative thereof, or a pharmaceutically acceptable salt of any of the foregoing, is administered daily. In some embodiments, about 10 mg to about 40 mg of Compound **I,** a deuterated derivative thereof, or a pharmaceutically acceptable salt of any of the foregoing, is administered daily. In some embodiments, about 30 mg to about 60 mg of Compound **I,** a deuterated derivative thereof, or a pharmaceutically acceptable salt of any of the foregoing, is administered daily. In some embodiments, about 50 mg to about 80 mg of Compound **I,** a deuterated derivative thereof, or a pharmaceutically acceptable salt of any of the foregoing, is administered daily.

In some embodiments, about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered daily. In some embodiments, about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered daily. In some embodiments, about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, is administered daily. In some embodiments, about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, is administered daily. In some embodiments, about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, is administered daily. In some embodiments, about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, is administered daily. In some embodiments, about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, is administered daily.

In some embodiments, the total daily dose of about 5 mg to about 100 mg (e.g., about 7 mg to about 90 mg, about 10 mg to about 80 mg, about 10 mg to about 40 mg, about 30 mg to about 60 mg, about 50 mg to about 80 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg) of Compound **I,** a deuterated derivative thereof, or a pharmaceutically acceptable salt of any of the foregoing, is administered as a single dose, once daily. In some embodiments, the total daily dose of about 5 mg to about 100 mg (e.g., about 7 mg to about 90 mg, about 10 mg to about 80 mg, about 10 mg to about 40 mg, about 30 mg to about 60 mg, about 50 mg to about 80 mg, about 5 mg, about 10 mg, about 15 mg, about 20 mg, about 25 mg, about 30 mg, about 35 mg, about 40 mg, about 45 mg, about 50 mg, about 55 mg, about 60 mg, about 65 mg, about 70 mg, about 75 mg, about 80 mg, about 85 mg, about 90 mg, about 95 mg, or about 100 mg) of Compound **I,** a deuterated derivative thereof, or a pharmaceutically acceptable salt of any of the foregoing, is administered in two doses.

In some embodiments, the total daily dose of about 0.1 mg to about 50 mg (e.g., about 0.25 mg to about 50 mg, about 0.25 mg to about 25 mg, about 1 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered as a single dose. In some embodiments, the total daily dose of about 0.1 mg to about 50 mg (e.g., about 0.25 mg to about 50 mg, about 0.25 mg to about 25 mg, about 1 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9 mg, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, is administered in two doses.

In some embodiments, the patient is heterozygous and has an F508del mutation on one allele and a mutation on the other allele selected from **Table 2:**

**Table 2: CFTR Mutations**

| **Mutation** | | | | |
|---|---|---|---|---|
| Q2X | L218X | Q525X | R792X | E1104X |
| S4X | Q220X | G542X | E822X | W1145X |
| W19X | Y275X | G550X | W882X | R1158X |
| G27X | C276X | Q552X | W846X | R1162X |
| Q39X | Q290X | R553X | Y849X | S 1196X |
| W57X | G330X | E585X | R851X | W1204X |
| E60X | W401X | G673X | Q890X | L1254X |
| R75X | Q414X | Q685X | S912X | S1255X |
| L88X | S434X | R709X | Y913X | W1282X |
| E92X | S466X | K710X | Q1042X | Q1313X |
| Q98X | S489X | Q715X | W1089X | Q1330X |
| Y122X | Q493X | L732X | Y1092X | E1371X |
| E193X | W496X | R764X | W1098X | Q1382X |
| W216X | C524X | R785X | R1102X | Q1411X |
| 185+1G→T | 711+5G→A | 1717-8G→A | 2622+1G→A | 3121-1G→A |
| 296+1G→A | 712-1G→T | 1717-1G→A | 2790-1G→C | 3500-2A→G |
| 296+1G→T | 1248+1G→A | 1811+1G→C | 3040G→C | 3600+2insT |
| 405+1G→A | 1249-1G→A | 1811+1.6kbA→G | (G970R) | 3850-1G→A |
| 405+3A→C | 1341+1G→A | 1811+1643G→T | 3120G→A | 4005+1G→A |
| 406-1G→A | 1525-2A→G | 1812-1G→A | 3120+1G→A | 4374+1G→T |
| 621+1G→T | 1525-1G→A | 1898+1G→A | 3121-2A→G | |
| 711+1G→T | | 1898+1G→7C | | |
| 182delT | 1078delT | 1677delTA | 2711delT | 3737delA |
| 306insA | 1119delA | 1782delA | 2732insA | 3791delC |
| 306delTAGA | 1138insG | 1824delA | 2869insG | 3821delT |
| 365-366insT | 1154insTC | 1833delT | 2896insAG | 3876delA |
| 394delTT | 1161delC | 2043delG | 2942insT | 3878delG |
| 442delA | 1213delT | 2143delT | 2957delT | 3905insT |
| 444delA | 1259insA | 2183AA→G | 3007delG | 4016insT |
| 457TAT→G | 1288insTA | 2184delA | 3028delA | 4021dupT |
| 541delC | 1343delG | 2184insA | 3171delC | 4022insT |
| 574delA | 1471delA | 2307insA | 3171insC | 4040delA |
| 663delT | 1497delGG | 2347delG | 3271delGG | 4279insA |
| 849delG | 1548delG | 2585delT | 3349insT | 4326delTC |
| 935delA | 1609del CA | 2594delGT | 3659delC | |
| CFTRdele1 | | CFTRdele16-17b | 1461ins4 | |
| CFTRdele2 | | CFTRdele17a,17b | 1924del7 | |
| CFTRdele2,3 | | CFTRdele 17a-18 | 2055del9→A | |
| CFTRdele2-4 | | CFTRdele19 | 2105-2117del13insAGAAA | |
| CFTRdele3-10,14b-16 | | CFTRdele 19-21 | 2372del8 | |
| CFTRdele4-7 | | CFTRdele21 | 2721del11 | |
| CFTRdele4-11 | | CFTRdele22-24 | 2991del32 | |
| CFTR50kbdel | | CFTRdele22,23 | 3667ins4 | |
| CFTRdup6b-10 | | 124del23bp | 4010del4 | |
| CFTRdele11 | | 602del14 | 4209TGTT-AA | |
| CFTRdele 13,14a | | 852del22 | | |
| CFTRdele 14b-17b | | 991del5 | | |
| A46D | V520F | Y569D | N1303K | |
| G85E | A559T | L1065P | | |
| R347P | R560T | R1066C | | |
| L467P | R560S | L1077P | | |
| I507del | A561E | M1101K | | |

In some embodiments, the disclosure also is directed to methods of treatment using isotope-labelled compounds of the afore-mentioned compounds or pharmaceutically acceptable salts thereof, wherein the formula and variables of such compounds and salts are each and independently as described above or any other embodiments described above, provided that one or more atoms therein have been replaced by an atom or atoms having an atomic mass or mass number which differs from the atomic mass or mass number of the atom which usually occurs naturally (isotope-labelled). Examples of isotopes which are commercially available and suitable for the disclosure include isotopes of hydrogen, carbon, nitrogen, oxygen, phosphorus, fluorine and chlorine, for example ²H, ³H, ¹³C, ¹⁴C, ¹⁵N, ¹⁸O, ¹⁷O, ³¹P, ³²P, ³⁵S, ¹⁸F and ³⁶Cl, respectively.

The isotope-labelled compounds and salts can be used in a number of beneficial ways. They can be suitable for medicaments and/or various types of assays, such as substrate tissue distribution assays. For example, tritium (³H)- and/or carbon-14 (¹⁴C)-labelled compounds are particularly useful for various types of assays, such as substrate tissue distribution assays, due to relatively simple preparation and excellent detectability. For example, deuterium (²H)-labelled ones are therapeutically useful with potential therapeutic advantages over the non-²H-labelled compounds. In general, deuterium (²H)-labelled compounds and salts can have higher metabolic stability as compared to those that are not isotope-labelled owing to the kinetic isotope effect described below. Higher metabolic stability translates directly into an increased in vivo half-life or lower dosages, which could be desired. The isotope-labelled compounds and salts can usually be prepared by carrying out the procedures disclosed in the synthesis schemes and the related description, in the example part and in the preparation part in the present text, replacing a non-isotope-labelled reactant by a readily available isotope-labelled reactant.

In some embodiments, the isotope-labelled compounds and salts are deuterium (²H)-labelled ones. In some specific embodiments, the isotope-labelled compounds and salts are deuterium (²H)-labelled, wherein one or more hydrogen atoms therein have been replaced by deuterium. In chemical structures, deuterium is represented as "²H" or "D."

When discovering and developing therapeutic agents, the person skilled in the art attempts to optimize pharmacokinetic parameters while retaining desirable in vitro properties. It may be reasonable to assume that many compounds with poor pharmacokinetic profiles are susceptible to oxidative metabolism.

The deuterium (²H)-labelled compounds and salts can modulate the oxidative metabolism of the compound by way of the primary kinetic isotope effect. The primary kinetic isotope effect is a change of the rate for a chemical reaction that results from exchange of isotopic nuclei, which in turn is caused by the change in ground state energies necessary for covalent bond formation after this isotopic exchange. Exchange of a heavier isotope usually results in a lowering of the ground state energy for a chemical bond and thus causes a reduction in the rate-limiting bond breakage. If the bond breakage occurs in or in the vicinity of a saddle-point region along the coordinate of a multi-product reaction, the product distribution ratios can be altered substantially. For explanation: if deuterium is bonded to a carbon atom at a non-exchangeable position, rate differences of k_{M/}k_{D} = 2-7 are typical. For a further discussion, see S. L. Harbeson and R. D. Tung, Deuterium In Drug Discovery and Development, Ann. Rep. Med. Chem. 2011, 46, 403-417, which is incorporated herein by reference.

The concentration of the isotope(s) (e.g., deuterium) incorporated into the isotope-labelled compounds and salt of the disclosure may be defined by the isotopic enrichment factor. The term "isotopic enrichment factor" as used herein means the ratio between the isotopic abundance and the natural abundance of a specified isotope. In some embodiments, if a substituent in a compound of the disclosure is denoted deuterium, such compound has an isotopic enrichment factor for each designated deuterium atom of at least 3500 (52.5% deuterium incorporation at each designated deuterium atom), at least 4000 (60% deuterium incorporation), at least 4500 (67.5% deuterium incorporation), at least 5000 (75% deuterium incorporation), at least 5500 (82.5% deuterium incorporation), at least 6000 (90% deuterium incorporation), at least 6333.3 (95% deuterium incorporation), at least 6466.7 (97% deuterium incorporation), at least 6600 (99% deuterium incorporation), or at least 6633.3 (99.5% deuterium incorporation).

### Combination Therapies

One aspect disclosed herein provides methods of treating CF and other CFTR-mediated diseases using Compound **I,** a deuterated derivative thereof, or a pharmaceutically acceptable salt of any of the foregoing, in combination with at least one additional active pharmaceutical ingredient (API).

Thus, in some embodiments, the invention provides methods of treating, lessening the severity of, or symptomatically treating cystic fibrosis in a patient comprising administering to the patient about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, alone or in combination with at least one additional active pharmaceutical ingredient, such as, e.g., a CFTR-modulating agent.

In some embodiments, the invention provides methods of treating, lessening the severity of, or symptomatically treating cystic fibrosis in a patient comprising administering to the patient, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, alone or in combination with at least one additional active pharmaceutical ingredient, such as, e.g., a CFTR-modulating agent.

In some embodiments, the invention provides methods of treating, lessening the severity of, or symptomatically treating cystic fibrosis in a patient comprising administering to the patient about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, of Compound **I,** a deuterated derivative thereof, or a pharmaceutically acceptable salt of any of the foregoing, alone or in combination with at least one additional active pharmaceutical ingredient, such as, e.g., a CFTR-modulating agent.

In some embodiments, the invention provides methods of treating, lessening the severity of, or symptomatically treating cystic fibrosis in a patient comprising administering to the patient about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, of Compound **I,** a deuterated derivative thereof, or a pharmaceutically acceptable salt of any of the foregoing, alone or in combination with at least one additional active pharmaceutical ingredient, such as, e.g., a CFTR-modulating agent.

In some embodiments, the invention provides methods of treating, lessening the severity of, or symptomatically treating cystic fibrosis in a patient comprising administering to the patient about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, of Compound **I,** a deuterated derivative thereof, or a pharmaceutically acceptable salt of any of the foregoing, alone or in combination with at least one additional active pharmaceutical ingredient, such as, e.g., a CFTR-modulating agent.

In some embodiments, the invention provides methods of treating, lessening the severity of, or symptomatically treating cystic fibrosis in a patient comprising administering to the patient about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, of Compound **I**, a deuterated derivative thereof, or a pharmaceutically acceptable salt of any of the foregoing, alone or in combination with at least one additional active pharmaceutical ingredient, such as, e.g., a CFTR-modulating agent.

In some embodiments, the invention provides methods of treating, lessening the severity of, or symptomatically treating cystic fibrosis in a patient comprising administering to the patient about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, alone or in combination with at least one additional active pharmaceutical ingredient, such as, e.g., a CFTR-modulating agent.

In some embodiments, the invention provides methods of treating, lessening the severity of, or symptomatically treating cystic fibrosis in a patient comprising administering to the patient about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, alone or in combination with at least one additional active pharmaceutical ingredient, such as, e.g., a CFTR-modulating agent.

In some embodiments, the invention provides methods of treating, lessening the severity of, or symptomatically treating cystic fibrosis in a patient comprising administering to the patient, about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, alone or in combination with at least one additional active pharmaceutical ingredient, such as, e.g., a CFTR-modulating agent.

In some embodiments, at least one additional API is selected from mucolytic agents, bronchodilators, antibiotics, anti-infective agents, anti-inflammatory agents, and nutritional agents.

In some embodiments, the additional agent is a mucolyte. Exemplary mucolytes useful herein include Pulmozyme^{®}.

In some embodiments, the additional agent is a bronchodilator. Exemplary bronchodilators include albuterol, metaprotenerol sulfate, pirbuterol acetate, salmeterol, and tetrabuline sulfate.

In some embodiments, the additional therapeutic agent is an antibiotic. Exemplary antibiotics useful herein include tobramycin, including tobramycin inhaled powder (TIP), azithromycin, aztreonam, including the aerosolized form of aztreonam, amikacin, including liposomal formulations thereof, ciprofloxacin, including formulations thereof suitable for administration by inhalation, levoflaxacin, including aerosolized formulations thereof, and combinations of two antibiotics, e.g., fosfomycin and tobramycin.

In some embodiments, the additional agent is an anti-inflammatory agent, i.e., an agent that can reduce the inflammation in the lungs. Exemplary such agents useful herein include ibuprofen, docosahexanoic acid (DHA), sildenafil, inhaled glutathione, pioglitazone, hydroxychloroquine, and simavastatin.

In some embodiments, the additional agent is a nutritional agent. Exemplary nutritional agents include pancrelipase (pancreating enzyme replacement), including Pancrease^{®}, Pancreacarb^{®}, Ultrase^{®}, or Creon^{®}, Liprotomase^{®} (formerly Trizytek^{®}), Aquadeks^{®}, and glutathione inhalation. In one embodiment, the additional nutritional agent is pancrelipase.

In some embodiments, at least one additional active pharmaceutical ingredient is selected from CFTR-modulating agents. In some embodiments, the CFTR-modulating agent is a CFTR corrector. In some embodiments, the CFTR-modulating agent is a CFTR potentiator enhancer/co-potentiator (for example, ASP-11). In some embodiments, the at least one additional active pharmaceutical ingredient is a CFTR amplifier. In some embodiments, the at least one additional active pharmaceutical ingredient is a CFTR readthrough agent. In some embodiments, the at least one additional active pharmaceutical ingredient is a CFTR nucleic acid therapy.

In some embodiments, the at least one additional active pharmaceutical ingredient is an ENaC inhibitor. In some embodiments, the at least one additional active pharmaceutical ingredient is a TMEM16A modulator. In some embodiments, the at least one additional active pharmaceutical ingredient is a GPR39 agonist.

In some embodiments, the at least one additional active pharmaceutical ingredient is chosen from (a) Compound **II,** deuterated derivatives of Compound **II** and pharmaceutically acceptable salts of Compound **II** and deuterated derivatives thereof; (b) Compound **III,** deuterated derivatives of Compound **III** and pharmaceutically acceptable salts of Compound **III** and deuterated derivatives thereof; (c) Compound **III-d,** and pharmaceutically acceptable salts of Compound **III-d;** (d) Compound **IV,** deuterated derivatives of Compound **IV** and pharmaceutically acceptable salts of Compound **IV** and deuterated derivatives thereof; (e) Compound **V,** deuterated derivatives of Compound **V** and pharmaceutically acceptable salts of Compound **V** and deuterated derivatives thereof; (f) Compound **VI,** deuterated derivatives of Compound **VI** and pharmaceutically acceptable salts of Compound **VI** and deuterated derivatives thereof; (g) Compound **VII,** deuterated derivatives of Compound **VII** and pharmaceutically acceptable salts of Compound **VII** and deuterated derivatives thereof; (h) Compound **VIII,** deuterated derivatives of Compound **VIII** and pharmaceutically acceptable salts of Compound **VIII** and deuterated derivatives thereof; (i) Compound **IX,** deuterated derivatives of Compound **IX** and pharmaceutically acceptable salts of Compound **IX** and deuterated derivatives thereof; and (j) Compound **X,** deuterated derivatives of Compound **X** and pharmaceutically acceptable salts of Compound **X** and deuterated derivatives thereof. Thus, in some embodiments, the combination therapies provided herein comprise administering Compound **I,** a deuterated derivative thereof, or a pharmaceutically acceptable salt of any of the foregoing; and at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof.

In some embodiments, the combination therapies provided herein comprise administering (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888.

In some embodiments, the combination therapies provided herein comprise administering (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888.

In some embodiments, the combination therapies provided herein comprise administering (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888.

In some embodiments, the combination therapies provided herein comprise administering (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888.

In some embodiments, the combination therapies provided herein comprise administering (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888.

In some embodiments, the combination therapies provided herein comprise administering (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888.

In some embodiments, the combination therapies provided herein comprise administering (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888.

In some embodiments, the combination therapies provided herein comprise administering (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888.

In some embodiments, the combination therapies provided herein comprise administering (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039.

In some embodiments, the combination therapies provided herein comprise administering (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039.

In some embodiments, the combination therapies provided herein comprise administering (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039.

In some embodiments, the combination therapies provided herein comprise administering (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039.

In some embodiments, the combination therapies provided herein comprise administering (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039.

In some embodiments, the combination therapies provided herein comprise administering (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039.

In some embodiments, the combination therapies provided herein comprise administering (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039.

In some embodiments, the combination therapies provided herein comprise administering (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; and (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039.

In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **II** and pharmaceutically acceptable salts and deuterated derivatives thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **III** and pharmaceutically acceptable salts and deuterated derivatives thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **III-d** and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **IV** and deuterated derivatives and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **V** and deuterated derivatives and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **VI** and deuterated derivatives and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **VII** and deuterated derivatives and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **VIII** and deuterated derivatives and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **IX** and deuterated derivatives and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **X** and deuterated derivatives and pharmaceutically acceptable salts thereof.

In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **II** and pharmaceutically acceptable salts and deuterated derivatives thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **IV** and deuterated derivatives and pharmaceutically acceptable salts thereof. In some embodiments, about 0.1 mg to about 50 mg (e.g., about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **V** and deuterated derivatives and pharmaceutically acceptable salts thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **VI** and deuterated derivatives and pharmaceutically acceptable salts thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **VII** and deuterated derivatives and pharmaceutically acceptable salts thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **VIII** and deuterated derivatives and pharmaceutically acceptable salts thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **IX** and deuterated derivatives and pharmaceutically acceptable salts thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **X** and deuterated derivatives and pharmaceutically acceptable salts thereof.

In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **III** and pharmaceutically acceptable salts and deuterated derivatives thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **III-d** and pharmaceutically acceptable salts thereof.

In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **II** and deuterated derivatives and pharmaceutically acceptable salts thereof, and at least one compound chosen from Compound **V** and pharmaceutically acceptable salts and deuterated derivatives thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I**, a deuterated derivative thereof, or a pharmaceutically acceptable salt of any of the foregoing, is administered in combination with at least one compound chosen from Compound **II** and deuterated derivatives and pharmaceutically acceptable salts thereof, and at least one compound chosen from Compound **VI** and deuterated derivatives and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **II** and deuterated derivatives and pharmaceutically acceptable salts thereof, and at least one compound chosen from Compound **VII** and deuterated derivatives and pharmaceutically acceptable salts thereof. In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in combination with at least one compound chosen from Compound **II** and deuterated derivatives and pharmaceutically acceptable salts thereof, and at least one compound chosen from Compound **VIII** and deuterated derivatives and pharmaceutically acceptable salts thereof.

In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **II** and deuterated derivatives and pharmaceutically acceptable salts thereof, and at least one compound chosen from Compound **V** and pharmaceutically acceptable salts and deuterated derivatives thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **II** and deuterated derivatives and pharmaceutically acceptable salts thereof, and at least one compound chosen from Compound **VI** and deuterated derivatives and pharmaceutically acceptable salts thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **II** and deuterated derivatives and pharmaceutically acceptable salts thereof, and at least one compound chosen from Compound **VII** and deuterated derivatives and pharmaceutically acceptable salts thereof. In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in combination with at least one compound chosen from Compound **II** and deuterated derivatives and pharmaceutically acceptable salts thereof, and at least one compound chosen from Compound **VIII** and deuterated derivatives and pharmaceutically acceptable salts thereof.

Each of Compound **I,** a deuterated derivative thereof, or a pharmaceutically acceptable salt of any of the foregoing, and compounds selected from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, independently can be administered once daily, twice daily, or three times daily.

In some embodiments, Compound **I,** a deuterated derivative thereof, or a pharmaceutically acceptable salt of any of the foregoing, is administered once daily. In some embodiments, Compound **I,** a deuterated derivative thereof, or a pharmaceutically acceptable salt of any of the foregoing, is administered twice daily. In some embodiments, Compound **I,** a deuterated derivative thereof, or a pharmaceutically acceptable salt of any of the foregoing, and at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof are administered once daily. In some embodiments, Compound **I,** a deuterated derivative thereof, or a pharmaceutically acceptable salt of any of the foregoing, and at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof are administered twice daily. In some embodiments, Compound **I,** a deuterated derivative thereof, or a pharmaceutically acceptable salt of any of the foregoing, and at least one compound chosen from Compound **II** and deuterated derivatives and pharmaceutically acceptable salts thereof are administered once daily. In some embodiments Compound **I,** a deuterated derivative thereof, or a pharmaceutically acceptable salt of any of the foregoing, and at least one compound chosen from Compound **II** and deuterated derivatives and pharmaceutically acceptable salts thereof are administered twice daily.

Compound **I,** a deuterated derivative thereof, or a pharmaceutically acceptable salt of any of the foregoing, and an additional therapeutic agent, can be administered in a single pharmaceutical composition or separate pharmaceutical compositions. Thus, (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, and (b) a compound selected from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof , can be administered in a single pharmaceutical composition comprising (a) and (b), or in separate pharmaceutical compositions wherein the first composition comprises (a) and the second composition comprises (b). Such pharmaceutical compositions can be administered once daily or multiple times daily, such as two or three times daily. As used herein, the phrase that a given amount of API (e.g., Compound **I,** a deuterated derivative thereof, or a pharmaceutically acceptable salt of any of the foregoing) is administered once or twice daily or per day means that said given amount is administered per dosing, which may occur once or twice daily.

Compound **I,** a deuterated derivative thereof, or a pharmaceutically acceptable salt of any of the foregoing, and an additional therapeutic agent, can be administered in a single pharmaceutical composition or separate pharmaceutical compositions. Thus, (a) about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, and (b) a compound selected from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, can be administered in a single pharmaceutical composition comprising (a) and (b), or in separate pharmaceutical compositions wherein the first composition comprises (a) and the second composition comprises (b). Such pharmaceutical compositions can be administered once daily or multiple times daily, such as two or three times daily. As used herein, the phrase that a given amount of API (e.g., Compound **I,** a deuterated derivative thereof, or a pharmaceutically acceptable salt of any of the foregoing) is administered once or twice daily or per day means that said given amount is administered per dosing, which may occur once or twice daily.

In some embodiments, about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, is administered in a first pharmaceutical composition; and at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof is administered in a second pharmaceutical composition.

In some embodiments, about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, is administered in a first pharmaceutical composition; and at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof is administered in a second pharmaceutical composition

Any suitable pharmaceutical compositions known in the art can be used for Compound **I,** Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof. Some exemplary pharmaceutical compositions for Compound **II** and its pharmaceutically acceptable salts can be found in WO 2011/119984 and WO 2014/014841. Some exemplary pharmaceutical compositions for Compound **III** and its pharmaceutically acceptable salts can be found in WO 2007/134279, WO 2010/019239, WO 2011/019413, WO 2012/027731, and WO 2013/130669, and some exemplary pharmaceutical compositions for Compound **III-d** and its pharmaceutically acceptable salts can be found in US 8,865,902, US 9,181,192, US 9,512,079, WO 2017/053455, and WO 2018/080591. Some exemplary pharmaceutical compositions for Compound **IV** and its pharmaceutically acceptable salts can be found in WO 2010/037066, WO 2011/127421, and WO 2014/071122. Some exemplary pharmaceutical compositions for Compound **V** and its pharmaceutically acceptable salts can be found in WO 2019/152940. Some exemplary pharmaceutical compositions for Compound **VI** and its pharmaceutically acceptable salts can be found in WO 2019/079760.

### Pharmaceutical Compositions

Another aspect of the invention provides a pharmaceutical composition comprising Compound **I,** a deuterated derivative thereof, or a pharmaceutically acceptable salt of any of the foregoing, and at least one pharmaceutically acceptable carrier.

In some embodiments, the disclosure provides a pharmaceutical composition comprising (a) about 5 mg to about 100 mg of Compound **I,** a deuterated derivative thereof, or a pharmaceutically acceptable salt of any of the foregoing, and (b) at least one pharmaceutically acceptable carrier. In some embodiments, the disclosure provides a pharmaceutical composition comprising (a) about 7 mg to about 90 mg of Compound **I,** a deuterated derivative thereof, or a pharmaceutically acceptable salt of any of the foregoing, and (b) at least one pharmaceutically acceptable carrier. In some embodiments, the disclosure provides a pharmaceutical composition comprising (a) about 10 mg to about 80 mg of Compound **I,** a deuterated derivative thereof, or a pharmaceutically acceptable salt of any of the foregoing, and (b) at least one pharmaceutically acceptable carrier. In some embodiments, the disclosure provides a pharmaceutical composition comprising (a) about 10 mg to about 40 mg of Compound **I,** a deuterated derivative thereof, or a pharmaceutically acceptable salt of any of the foregoing, and (b) at least one pharmaceutically acceptable carrier. In some embodiments, the disclosure provides a pharmaceutical composition comprising (a) about 30 mg to about 60 mg of Compound **I**, a deuterated derivative thereof, or a pharmaceutically acceptable salt of any of the foregoing, and (b) at least one pharmaceutically acceptable carrier. In some embodiments, the disclosure provides a pharmaceutical composition comprising (a) about 50 mg to about 80 mg of Compound **I,** a deuterated derivative thereof, or a pharmaceutically acceptable salt of any of the foregoing, and (b) at least one pharmaceutically acceptable carrier.

In some embodiments, the disclosure provides a pharmaceutical composition comprising (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, and (b) at least one pharmaceutically acceptable carrier.

In some embodiments, the disclosure provides a pharmaceutical composition comprising (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, and (b) at least one pharmaceutically acceptable carrier.

In some embodiments, the disclosure provides a pharmaceutical composition comprising (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, and (b) at least one pharmaceutically acceptable carrier.

In some embodiments, the disclosure provides a pharmaceutical composition comprising (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, and (b) at least one pharmaceutically acceptable carrier.

In some embodiments, the disclosure provides a pharmaceutical composition comprising (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, and (b) at least one pharmaceutically acceptable carrier.

In some embodiments, the disclosure provides a pharmaceutical composition comprising (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, and (b) at least one pharmaceutically acceptable carrier.

In some embodiments, the disclosure provides a pharmaceutical composition comprising (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, and (b) at least one pharmaceutically acceptable carrier.

In some embodiments, the invention provides pharmaceutical compositions comprising about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, and optionally at least one additional active pharmaceutical ingredient. In some embodiments, the at least one additional active pharmaceutical ingredient is a CFTR modulator. In some embodiments, the at least one additional active pharmaceutical ingredient is a CFTR corrector. In some embodiments, the at least one additional active pharmaceutical ingredient is a CFTR potentiator. In some embodiments, the at least one additional active pharmaceutical ingredient is a compound that enhances CFTR potentiation, i.e., a CFTR potentiator enhancer/co-potentiator. In some embodiments, the at least one additional active pharmaceutical ingredient is a CFTR amplifier. In some embodiments, the at least one additional active pharmaceutical ingredient is a CFTR readthrough agent. In some embodiments, the at least one additional active pharmaceutical ingredient is a CFTR nucleic acid therapy. In some embodiments, the at least one additional active pharmaceutical ingredient is an ENaC inhibitor. In some embodiments, the at least one additional active pharmaceutical ingredient is a TMEM16A modulator. In some embodiments, the at least one additional active pharmaceutical ingredient is a GPR39 agonist. In some embodiments, the pharmaceutical composition comprises Compound **I,** a deuterated derivative thereof, or a pharmaceutically acceptable salt of any of the foregoing, and at least one additional active pharmaceutical ingredient, which is a CFTR corrector. In some embodiments, the pharmaceutical composition comprises Compound **I,** a deuterated derivative thereof, or a pharmaceutically acceptable salt of any of the foregoing, and at least two additional active pharmaceutical ingredients, each of which is a CFTR corrector.

In some embodiments, the invention provides pharmaceutical compositions comprising about 0.1 mg to about 50 mg (e.g., about 0.25 mg to about 50 mg, about 0.25 mg to about 25 mg, about 0.5 mg to about 15 mg, about 1.5 mg to about 14 mg, about 4 mg to about 13 mg, about 8 mg to about 13 mg, about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, and optionally at least one additional active pharmaceutical ingredient. In some embodiments, the at least one additional active pharmaceutical ingredient is a CFTR modulator. In some embodiments, the at least one additional active pharmaceutical ingredient is a CFTR corrector. In some embodiments, the at least one additional active pharmaceutical ingredient is a CFTR potentiator. In some embodiments, the at least one additional active pharmaceutical ingredient is a compound that enhances CFTR potentiation, i.e., a CFTR potentiator enhancer/co-potentiator. In some embodiments, the at least one additional active pharmaceutical ingredient is a CFTR amplifier. In some embodiments, the at least one additional active pharmaceutical ingredient is a CFTR readthrough agent. In some embodiments, the at least one additional active pharmaceutical ingredient is a CFTR nucleic acid therapy. In some embodiments, the at least one additional active pharmaceutical ingredient is an ENaC inhibitor. In some embodiments, the at least one additional active pharmaceutical ingredient is a TMEM16A modulator. In some embodiments, the at least one additional active pharmaceutical ingredient is a GPR39 agonist. In some embodiments, the pharmaceutical composition comprises Compound **I,** a deuterated derivative thereof, or a pharmaceutically acceptable salt of any of the foregoing, and at least one additional active pharmaceutical ingredient, which is a CFTR corrector. In some embodiments, the pharmaceutical composition comprises Compound **I,** a deuterated derivative thereof, or a pharmaceutically acceptable salt of any of the foregoing, and at least two additional active pharmaceutical ingredients, each of which is a CFTR corrector.

In some embodiments, the invention provides a pharmaceutical composition comprising (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier. In some embodiments, the invention provides a pharmaceutical composition comprising (a) Compound **I,** a deuterated derivative thereof, or a pharmaceutically acceptable salt of any of the foregoing, (b) at least two compounds chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier.

In some embodiments, the invention provides a pharmaceutical composition comprising (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier. In some embodiments, the invention provides a pharmaceutical composition comprising (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) at least two compounds chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier.

In some embodiments, the invention provides a pharmaceutical composition comprising (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier. In some embodiments, the invention provides a pharmaceutical composition comprising (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) at least two compounds chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier.

In some embodiments, the invention provides a pharmaceutical composition comprising (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier. In some embodiments, the invention provides a pharmaceutical composition comprising (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) at least two compounds chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier.

In some embodiments, the invention provides a pharmaceutical composition comprising (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier. In some embodiments, the invention provides a pharmaceutical composition comprising (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) at least two compounds chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier.

In some embodiments, the invention provides a pharmaceutical composition comprising (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier. In some embodiments, the invention provides a pharmaceutical composition comprising (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) at least two compounds chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier.

In some embodiments, the invention provides a pharmaceutical composition comprising (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier. In some embodiments, the invention provides a pharmaceutical composition comprising (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) at least two compounds chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier.

In some embodiments, the invention provides a pharmaceutical composition comprising (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier. In some embodiments, the invention provides a pharmaceutical composition comprising (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) at least two compounds chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (c) at least one pharmaceutically acceptable carrier.

In some embodiments, the invention provides a pharmaceutical composition comprising (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) at least one compound chosen from Compound **II** and deuterated derivatives and pharmaceutically acceptable salts thereof, and (c) at least one pharmaceutically acceptable carrier.

In some embodiments, the invention provides a pharmaceutical composition comprising (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) at least one compound chosen from Compound **II** and deuterated derivatives and pharmaceutically acceptable salts thereof, and (c) at least one pharmaceutically acceptable carrier.

In some embodiments, the invention provides a pharmaceutical composition comprising (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) at least one compound chosen from Compound **II** and deuterated derivatives and pharmaceutically acceptable salts thereof, and (c) at least one pharmaceutically acceptable carrier.

In some embodiments, the invention provides a pharmaceutical composition comprising (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) at least one compound chosen from Compound **II** and deuterated derivatives and pharmaceutically acceptable salts thereof, and (c) at least one pharmaceutically acceptable carrier.

In some embodiments, the invention provides a pharmaceutical composition comprising (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) at least one compound chosen from Compound **II** and deuterated derivatives and pharmaceutically acceptable salts thereof, and (c) at least one pharmaceutically acceptable carrier.

In some embodiments, the invention provides a pharmaceutical composition comprising (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) at least one compound chosen from Compound **II** and deuterated derivatives and pharmaceutically acceptable salts thereof, and (c) at least one pharmaceutically acceptable carrier.

In some embodiments, the invention provides a pharmaceutical composition comprising (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) at least one compound chosen from Compound **II** and deuterated derivatives and pharmaceutically acceptable salts thereof, and (c) at least one pharmaceutically acceptable carrier.

In some embodiments, the invention provides a pharmaceutical composition comprising (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) at least one compound chosen from Compound **II** and deuterated derivatives and pharmaceutically acceptable salts thereof, and (c) at least one pharmaceutically acceptable carrier.

In some embodiments, the disclosure provides a pharmaceutical composition comprising (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) at least one compound chosen from Compound **II,** and deuterated derivatives and pharmaceutically acceptable salts thereof, (c) at least one compound chosen from Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (d) at least one pharmaceutically acceptable carrier.

In some embodiments, the disclosure provides a pharmaceutical composition comprising (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) at least one compound chosen from Compound **II,** and deuterated derivatives and pharmaceutically acceptable salts thereof, (c) at least one compound chosen from Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (d) at least one pharmaceutically acceptable carrier.

In some embodiments, the disclosure provides a pharmaceutical composition comprising (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) at least one compound chosen from Compound **II,** and deuterated derivatives and pharmaceutically acceptable salts thereof, (c) at least one compound chosen from Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (d) at least one pharmaceutically acceptable carrier.

In some embodiments, the disclosure provides a pharmaceutical composition comprising (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) at least one compound chosen from Compound **II,** and deuterated derivatives and pharmaceutically acceptable salts thereof, (c) at least one compound chosen from Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (d) at least one pharmaceutically acceptable carrier.

In some embodiments, the disclosure provides a pharmaceutical composition comprising (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) at least one compound chosen from Compound **II,** and deuterated derivatives and pharmaceutically acceptable salts thereof, (c) at least one compound chosen from Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (d) at least one pharmaceutically acceptable carrier.

In some embodiments, the disclosure provides a pharmaceutical composition comprising (a) about 1.5 mg to about 14 mg (e.g., about 1.5 mg, about 4 mg, about 6.5 mg, about 9 mg, about 11.5 mg, or about 14 mg) of Compound **I,** a deuterated derivative thereof, or a pharmaceutically acceptable salt of any of the foregoing, (b) at least one compound chosen from Compound **II,** and deuterated derivatives and pharmaceutically acceptable salts thereof, (c) at least one compound chosen from Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (d) at least one pharmaceutically acceptable carrier.

In some embodiments, the disclosure provides a pharmaceutical composition comprising (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) at least one compound chosen from Compound **II,** and deuterated derivatives and pharmaceutically acceptable salts thereof, (c) at least one compound chosen from Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (d) at least one pharmaceutically acceptable carrier.

In some embodiments, the disclosure provides a pharmaceutical composition comprising (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) at least one compound chosen from Compound **II,** and deuterated derivatives and pharmaceutically acceptable salts thereof, (c) at least one compound chosen from Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof, and (d) at least one pharmaceutically acceptable carrier.

In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888; and (d) at least one pharmaceutically acceptable carrier.

In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888; and (d) at least one pharmaceutically acceptable carrier.

In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888; and (d) at least one pharmaceutically acceptable carrier.

In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888; and (d) at least one pharmaceutically acceptable carrier.

In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound I, a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888; and (d) at least one pharmaceutically acceptable carrier.

In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888; and (d) at least one pharmaceutically acceptable carrier.

In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888; and (d) at least one pharmaceutically acceptable carrier.

In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from compounds disclosed in WO 2016/105485, United States Patent Application Publication No. 2016-0120841, United States Patent Application Publication No. 2017-0101405, WO 2017/009804, WO 2018/065921, WO 2017/062581; WO 2022/076618; WO 2022/076620; WO 2022/076621; WO 2022/076622; WO 2022/076624; WO 2022/076625; WO 2022/076626; WO 2022/076627; WO 2022/076628; WO 2022/076629; United States Provisional Patent Application Nos. 63/328,097 and 63/393,405; Phuan, P.-W. et al. J. Cyst. Fibros. 2018, 17 (5), 595-606; Pedemonte, N. et al. Sci. Adv. 2020, 6 (8), eaay9669; Phuan, P.-W. et al. Sci. Rep. 2019, 9 (1), 17640; Bose, S. et al. J. Cyst. Fibros. 2020, 19 Suppl 1, S25-S32; Crawford, D.K. J. Pharmacol. Exp. Ther. 2020, 374 (2), 264-272; Brasell, E.J. et al. PLoS One 2019, 14 (12), e0223954; Smith, N.J, Solovay, C.F., Pharm. Pat. Anal. 2017, 6 (4), 179-188; Kunzelmann, K. et al., Front. Pharmacol. 2019, 10, 3; or Son, J.-H. et al., Eur. J. of Med. Chem. 2020, 112888; and (d) at least one pharmaceutically acceptable carrier.

In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039; and (d) at least one pharmaceutically acceptable carrier.

In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound I, a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039; and (d) at least one pharmaceutically acceptable carrier.

In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039; and (d) at least one pharmaceutically acceptable carrier.

In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from PTI-428, ASP-11, ABBV-2222, ABBV-2851, GLPG2737,
ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039; and (d) at least one pharmaceutically acceptable carrier.

In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from PTI-428, ASP-11, ABBV-2222, ABBV-2851, GLPG2737,
ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039; and (d) at least one pharmaceutically acceptable carrier.

In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from PTI-428, ASP-11, ABBV-2222, ABBV-2851, GLPG2737,
ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039; and (d) at least one pharmaceutically acceptable carrier.

In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing; (b) optionally at least one compound chosen from Compound **II,** Compound **III,** Compound **III-d**, Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from PTI-428, ASP-11, ABBV-2222, ABBV-2851, GLPG2737,
ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039; and (d) at least one pharmaceutically acceptable carrier.

In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing; (b) optionally at least one compound chosen from Compound **II,** Compound **III,** Compound **III-d,** Compound **IV,** Compound **V,** Compound **VI,** Compound **VII,** Compound **VIII,** Compound **IX,** Compound **X,** and deuterated derivatives and pharmaceutically acceptable salts thereof; (c) optionally at least one compound chosen from PTI-428, ASP-11, ABBV-2222, ABBV-2851, GLPG2737, ABBV-3221, ABBV-3748, ABBV-3903, ABBV-119, FDL-169, ARN5562, ARN21586, ARN22081, ARN22652, ARN23765, ARN23766, PTI-801, FDL-176, PTI-808, GLPG1837/ABBV-974, GLPG2451/ABBV-2451, QBW251 (Icenticaftor), GLPG3067/ABBV-3067 (Navocaftor), ABBV-191, ELX-02, MRT5005, Lunar-CF, RCT223, amiloride, ETD001, CF552, GS-9411, GS-5737, P-1037 (VX-371), P-1055 (VX-551), AZD5634, SPX-101, Ionis-ENaC-2.5 Rx, BI 1265162, ARO-ENaC1001, ETD002, and DS-1039; and (d) at least one pharmaceutically acceptable carrier.

In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **III,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **III-d,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **IV,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **V,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **VI,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg of (e.g., about 7 mg to about 90 mg, about 10 mg to about 80 mg, about 10 mg to about 40 mg, about 30 mg to about 60 mg, or about 50 mg to about 80 mg) of Compound **I**, a deuterated derivative thereof, or a pharmaceutically acceptable salt of any of the foregoing, (b) Compound **VII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg of (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **VIII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **IX,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **X,** a deuterated derivative thereof, or a pharmaceutically acceptable salt of any of the foregoing, and (c) at least one pharmaceutically acceptable carrier.

In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **II**, a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **III,** a deuterated derivative thereof, or a pharmaceutically acceptable salt of any of the foregoing, and (c) at least one pharmaceutically acceptable carrier. In some embodiments, the pharmaceutical compositions provided herein comprise (a) a about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **III-d,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **IV,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **V,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **VI,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **VII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **VIII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **IX,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **X,** a deuterated derivative thereof, or a pharmaceutically acceptable salt of any of the foregoing, and (c) at least one pharmaceutically acceptable carrier.

In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **III,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **III-d,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **IV,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **V,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **VI,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **VII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **VIII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **IX,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **X,** a deuterated derivative thereof, or a pharmaceutically acceptable salt of any of the foregoing, and (c) at least one pharmaceutically acceptable carrier.

In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **III,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **III-d,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **IV,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **V,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **VI,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **VII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **VIII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **IX,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **X,** a deuterated derivative thereof, or a pharmaceutically acceptable salt of any of the foregoing, and (c) at least one pharmaceutically acceptable carrier.

In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **III,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **III-d,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **IV,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **V,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **VI,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **VII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **VIII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **IX,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **X,** a deuterated derivative thereof, or a pharmaceutically acceptable salt of any of the foregoing, and (c) at least one pharmaceutically acceptable carrier.

In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **III,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **III-d,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **IV,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **V,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **VI,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **VII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **VIII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **IX,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **X,** a deuterated derivative thereof, or a pharmaceutically acceptable salt of any of the foregoing, and (c) at least one pharmaceutically acceptable carrier.

In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I**, a deuterated derivative thereof, or a pharmaceutically acceptable salt of any of the foregoing, (b) Compound **III,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **III-d,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **IV,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **V,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **VI,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **VII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **VIII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **IX,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I**, a deuterated derivative thereof, or a pharmaceutically acceptable salt of any of the foregoing, (b) Compound **X,** a deuterated derivative thereof, or a pharmaceutically acceptable salt of any of the foregoing, and (c) at least one pharmaceutically acceptable carrier.

In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **III,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **III-d,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **IV,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **V,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **VI,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **VII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **VIII,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **IX,** a deuterated derivative thereof, or a pharmaceutically acceptable salt of any of the foregoing, and (c) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **X,** a deuterated derivative thereof, or a pharmaceutically acceptable salt of any of the foregoing, and (c) at least one pharmaceutically acceptable carrier.

In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **V,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VI,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VII,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 5 mg to about 100 mg (e.g., about 7 mg to about 90 mg, about 10 mg to about 80 mg, about 10 mg to about 40 mg, about 30 mg to about 60 mg, or about 50 mg to about 80 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VIII,** a deuterated derivative thereof, or a pharmaceutically acceptable salt of any of the foregoing, and (d) at least one pharmaceutically acceptable carrier.

In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **II**, a deuterated derivative thereof, or a pharmaceutically acceptable salt of any of the foregoing, (c) Compound **V,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VI,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VII,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.1 mg to about 50 mg (e.g., about 0.1 mg, about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VIII,** a deuterated derivative thereof, or a pharmaceutically acceptable salt of any of the foregoing, and (d) at least one pharmaceutically acceptable carrier.

In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **V,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I**, a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VI,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VII,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.25 mg to about 50 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VIII,** a deuterated derivative thereof, or a pharmaceutically acceptable salt of any of the foregoing, and (d) at least one pharmaceutically acceptable carrier.

In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **V,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **II**, a deuterated derivative thereof, or a pharmaceutically acceptable salt of any of the foregoing, (c) Compound **VI,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **II**, a deuterated derivative thereof, or a pharmaceutically acceptable salt of any of the foregoing, (c) Compound **VII,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 0.25 mg to about 25 mg (e.g., about 0.25 mg, about 2.5 mg, about 5 mg, about 7.5 mg, about 10 mg, about 12.5 mg, about 15 mg, about 17.5 mg, about 20 mg, about 22.5 mg, or about 25 mg) of Compound **I,** a deuterated derivative thereof, or a pharmaceutically acceptable salt of any of the foregoing, (b) Compound **II**, a deuterated derivative thereof, or a pharmaceutically acceptable salt of any of the foregoing, (c) Compound **VIII,** a deuterated derivative thereof, or a pharmaceutically acceptable salt of any of the foregoing, and (d) at least one pharmaceutically acceptable carrier.

In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **V,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VI,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **II**, a deuterated derivative thereof, or a pharmaceutically acceptable salt of any of the foregoing, (c) Compound **VII,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 1 mg to about 15 mg (e.g., about 1 mg, about 2.5 mg, about 5 mg, about 7.5 mg, about 10 mg, about 12.5 mg, about 15 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VIII,** a deuterated derivative thereof, or a pharmaceutically acceptable salt of any of the foregoing, and (d) at least one pharmaceutically acceptable carrier.

In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **V,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VI,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **II**, a deuterated derivative thereof, or a pharmaceutically acceptable salt of any of the foregoing, (c) Compound **VII,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 1.5 mg to about 14 mg (e.g., about 1.5 mg, about 2.5 mg, about 5 mg, about 7.5 mg, about 10 mg, about 12.5 mg, about 14 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VIII,** a deuterated derivative thereof, or a pharmaceutically acceptable salt of any of the foregoing, and (d) at least one pharmaceutically acceptable carrier.

In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **V,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VI,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VII,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 4 mg to about 13 mg (e.g., about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VIII,** a deuterated derivative thereof, or a pharmaceutically acceptable salt of any of the foregoing, and (d) at least one pharmaceutically acceptable carrier.

In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **V,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VI,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VII,** a deuterated derivative thereof, or a pharmaceutically acceptable salt of any of the foregoing, and (d) at least one pharmaceutically acceptable carrier. In some embodiments, the pharmaceutical compositions provided herein comprise (a) about 8 mg to about 13 mg (e.g., about 8 mg, about 8.5 mg, about 9, about 9.5 mg, about 10 mg, about 10.5 mg, about 11 mg, about 11.5 mg, about 12 mg, about 12.5 mg, or about 13 mg) of Compound **I,** a deuterated derivative thereof, or a pharmaceutically acceptable salt of any of the foregoing, (b) Compound **II,** a deuterated derivative thereof, or a pharmaceutically acceptable salt of any of the foregoing, (c) Compound **VIII,** a deuterated derivative thereof, or a pharmaceutically acceptable salt of any of the foregoing, and (d) at least one pharmaceutically acceptable carrier.

Any pharmaceutical composition disclosed herein may comprise at least one pharmaceutically acceptable carrier. In some embodiments, the at least one pharmaceutically acceptable carrier is chosen from pharmaceutically acceptable vehicles and pharmaceutically acceptable adjuvants. In some embodiments, the at least one pharmaceutically acceptable is chosen from pharmaceutically acceptable fillers, disintegrants, surfactants, binders, and lubricants.

The pharmaceutical compositions described herein are useful for treating cystic fibrosis and other CFTR-mediated diseases.

As described above, pharmaceutical compositions disclosed herein may optionally further comprise at least one pharmaceutically acceptable carrier. The at least one pharmaceutically acceptable carrier may be chosen from adjuvants and vehicles. The at least one pharmaceutically acceptable carrier, as used herein, includes any and all solvents, diluents, other liquid vehicles, dispersion aids, suspension aids, surface active agents, isotonic agents, thickening agents, emulsifying agents, preservatives, solid binders, and lubricants, as suited to the particular dosage form desired. Remington: The Science and Practice of Pharmacy, 21st edition, 2005, ed. D.B. Troy, Lippincott Williams & Wilkins, Philadelphia, and Encyclopedia of Pharmaceutical Technology, eds. J. Swarbrick and J. C. Boylan, 1988-1999, Marcel Dekker, New York discloses various carriers used in formulating pharmaceutical compositions and known techniques for the preparation thereof. Except insofar as any conventional carrier is incompatible with the compounds of this disclosure, such as by producing any undesirable biological effect or otherwise interacting in a deleterious manner with any other component(s) of the pharmaceutical composition, its use is contemplated to be within the scope of this disclosure. Non-limiting examples of suitable pharmaceutically acceptable carriers include, but are not limited to, ion exchangers, alumina, aluminum stearate, lecithin, serum proteins (such as human serum albumin), buffer substances (such as phosphates, glycine, sorbic acid, and potassium sorbate), partial glyceride mixtures of saturated vegetable fatty acids, water, salts, and electrolytes (such as protamine sulfate, disodium hydrogen phosphate, potassium hydrogen phosphate, sodium chloride, and zinc salts), colloidal silica, magnesium trisilicate, polyvinyl pyrrolidone, polyacrylates, waxes, polyethylene-polyoxypropylene-block polymers, wool fat, sugars (such as lactose, glucose and sucrose), starches (such as corn starch and potato starch), cellulose and its derivatives (such as sodium carboxymethyl cellulose, ethyl cellulose and cellulose acetate), powdered tragacanth, malt, gelatin, talc, excipients (such as cocoa butter and suppository waxes), oils (such as peanut oil, cottonseed oil, safflower oil, sesame oil, olive oil, corn oil and soybean oil), glycols (such as propylene glycol and polyethylene glycol), esters (such as ethyl oleate and ethyl laurate), agar, buffering agents (such as magnesium hydroxide and aluminum hydroxide), alginic acid, pyrogen-free water, isotonic saline, Ringer's solution, ethyl alcohol, phosphate buffer solutions, non-toxic compatible lubricants (such as sodium lauryl sulfate and magnesium stearate), coloring agents, releasing agents, coating agents, sweetening agents, flavoring agents, perfuming agents, preservatives, and antioxidants.

### Examples

### Abbreviations

AE: adverse event
AUC: area under the concentration versus time curve
AUC_{0-∞}: AUC from the time of dosing extrapolated to infinity
AUC₀₋₂₄ₕ: AUC from the time of dosing to 24 hours
AUC₀₋ₜₗₐₛₜ: AUC from the time of dosing to the last measurable concentration
Boc anhydride ((Boc)₂O): Di-*tert*-butyl dicarbonate
Boc: *tert*-Butyloxycarbonyl
Cmax: maximum observed concentration
DCE: 1,2-Dichloroethane
DDI: drug-drug interaction
DIAD: Diisopropyl azodicarboxylate
DMSO: Dimethyl sulfoxide
DMSO-*d6*: Dimethyl sulfoxide-*d6*
EtOAc: Ethyl acetate
ESI-MS: Electrospray ionization mass spectrometry
FIH: first-in-human (study)
HBE: human bronchial epithelial (cells)
HPLC: High performance liquid chromatography
LC: Liquid chromatography
MS: Mass spectrometry
n: number of subjects
NMR: Nuclear magnetic resonance
NOAEL: no-observed-adverse-effect level
PD: pharmacodynamic, pharmacodynamics
Pd/C: Palladium on carbon
PK: pharmacokinetic, pharmacokinetics
q12h: every 12 hours
qd: once daily
RT or rt: Room temperature
TBD: to be determined
TFA: Trifluoroacetic acid
UPLC: Ultra Performance Liquid Chromatography

### Example 1: Preparation of (6R,12R)-17-amino-12-methyl-6,15-bis(trifluoromethyl)-13,19-dioxa-3,4,18-triazatricyclo[12.3.1.12,5]nonadeca-1(18),2,4,14,16-pentaen-6-ol

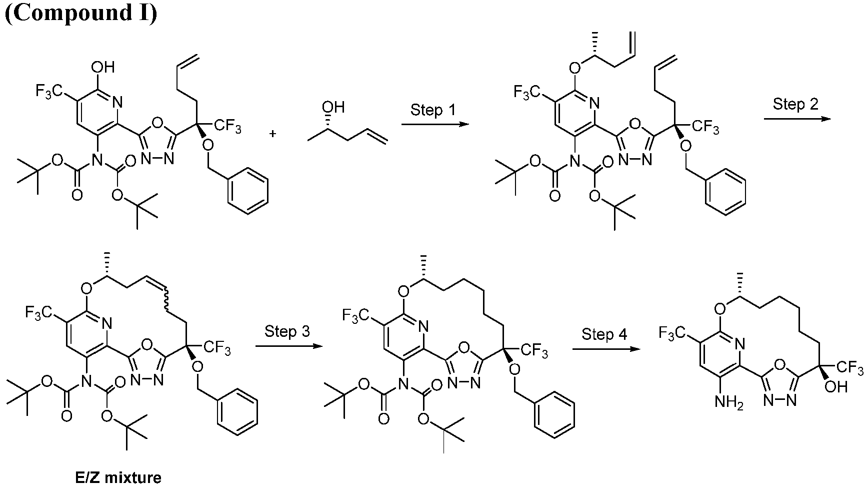

### Step 1: tert-Butyl N-[2-[5-[(1R)-1-benzyloxy-1-(trifluoromethyl)pent-4-enyl]-1,3,4-oxadiazol-2-yl]-6-[(1R)-1-methylbut-3-enoxy]-5-(trifluoromethyl)-3-pyridyl]-N-tert-butoxycarbonyl-carbamate

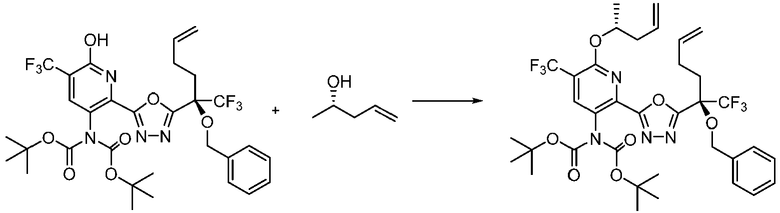

Dissolved *tert*-butyl *N*-[2-[5-[(1*R*)-1-benzyloxy-1-(trifluoromethyl)pent-4-enyl]-1,3,4-oxadiazol-2-yl]-6-hydroxy-5-(trifluoromethyl)-3-pyridyl]-*N*-*tert-*butoxycarbonyl-carbamate (159.3 g, 231.3 mmol) and triphenylphosphine (72.9 g, 277.9 mmol) in toluene (1 L), then added (2S)-pent-4-en-2-ol (28.7 mL, 278.9 mmol). Heated this mixture to 45 °C, then added DIAD (58.3 mL, 296.1 mmol) (exotherm) slowly over 40 min. For the next approximately 2 h, the mixture was cooled to room temperature. During this cooling period, after the first 10 minutes, triphenylphosphine (6.07 g, 23.14 mmol) was added. After a further 1 h, additional triphenylphosphine (3.04 g, 11.59 mmol) was added. After a further 23 min, DIAD (2.24 mL, 11.57 mmol) was added. After the ~2 h cooling to room temperature period, the mixture was cooled to 15 °C, and seed crystals of DIAD-triphenylphosphine oxide complex were added which caused precipitation to occur, then added 1000 mL heptane. Stored the mixture at -20 °C for 3 days. Filtered out and discarded the precipitate and concentrated the filtrate to give a red residue/oil. Dissolved the residue in 613 mL heptane at 45 °C, then cooled to 0 °C, seeded with DIAD-triphenylphosphine oxide complex, stirred at 0 °C for 30 min, then filtered the solution. The filtrate was concentrated to a smaller volume, then loaded onto a 1.5 kg silica gel column (column volume = 2400 mL, flow rate = 600 mL/min). Ran a gradient of 1% to 6% EtOAc in hexanes over 32 minutes (8 column volumes), then held at 6% EtOAc in hexanes until the product finished eluting which gave *tert*-butyl *N*-[2-[5-[(1R)-1-benzyloxy-1-(trifluoromethyl)pent-4-enyl]-1,3,4-oxadiazol-2-yl]-6-[(1*R*)-1-methylbut-3-enoxy]-5-(trifluoromethyl)-3-pyridyl]-*N*-*tert*-butoxycarbonyl-carbamate (163.5 g, 93%). ¹H NMR (400 MHz, Chloroform-d) δ 7.82 (s, 1H), 7.43 - 7.27 (m, 5H), 5.88 - 5.69 (m, 2H), 5.35 (h, *J =* 6.2 Hz, 1H), 5.16 - 4.94 (m, 4H), 4.81 (d, *J =* 10.7 Hz, 1H), 4.63 (d, *J =* 10.7 Hz, 1H), 2.58 - 2.15 (m, 6H), 1.42 (s, 18H), 1.36 (d, *J =* 6.2 Hz, 3H) ppm. ESI-MS *m*/*z* calc. 756.2958, found 757.3 (M+1)⁺; Retention time: 4.0 minutes. Final purity was determined by reversed phase UPLC using an Acquity UPLC BEH C₁₈ column (50 × 2.1 mm, 1.7 µm particle) made by Waters (pn: 186002350), and a dual gradient run from 1 - 99% mobile phase B over 4.5 minutes. Mobile phase A = water (0.05 % CF₃CO₂H). Mobile phase B = acetonitrile (0.035 % CF₃CO₂H). Flow rate = 1.2 mL/min, injection volume = 1.5 µL, and column temperature = 60°C.

### Step 2: tert-Butyl N-[(6R,12R)-6-benzyloxy-12-methyl-6,15-bis(trifluoromethyl)-13,19-dioxa-3,4,18-triazatricyclo[12.3.1.12,5]nonadeca-1(18),2,4,9,14,16-hexaen-17-yl]-N-tert-butoxycarbonyl-carbamate (E/Z mixture)

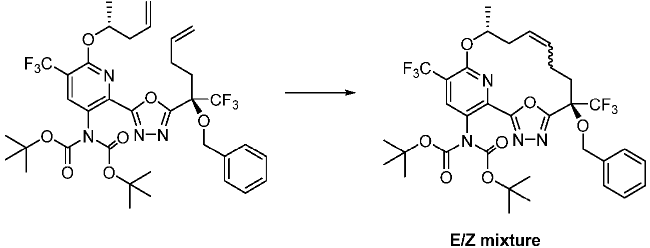

The following reaction was run, split equally between two, 12 L reaction flasks run in parallel. Mechanical stirring was employed, and reactions were subjected to a constant nitrogen gas purge using a course porosity gas dispersion tube. To each flask was added tert-butyl *N*-[2-[5-[(1*R*)-1-benzyloxy-1-(trifluoromethyl)pent-4-enyl]-1,3,4-oxadiazol-2-y1]*-*6*-*[(1*R*)*-*1-methylbut-3-enoxy]-5-(trifluoromethyl)-3-pyridyl]-*N*-*tert-*butoxycarbonyl-carbamate (54 g, 71.36 mmol in each flask) dissolved in DCE (8 L in each flask) and both flasks were strongly purged with nitrogen at room temperature. Both flasks were heated to 62 °C and Grubbs 1^{st} Generation Catalyst (9 g, 10.94 mmol in each flask) was added to each reaction and stirred at 400 rpm while setting an internal temperature control to 75 °C with strong nitrogen purging (both reactions reached ~75 °C after approximately 20 min). After 5 h 15 min, the internal temperature control was set to 45 °C. After approximately 2 h, 2-sulfanylpyridine-3-carboxylic acid (11 g, 70.89 mmol in each flask) was added to each flask followed by triethylamine (10 mL, 71.75 mmol in each flask). On completion of addition, the nitrogen purge was turned off and both reaction flasks were stirred at 45 °C open to air overnight. The reactions were then removed from heat and 130 g of silica gel was added to each reaction and each was stirred at room temperature. After approximately 2 h, the green mixtures were combined and filtered over Celite then concentrated by rotary evaporation at 43 °C. The obtained residue was dissolved in dichloromethane/heptane 1:1 (400 mL) and the formed orange solid was removed by filtration. The greenish mother liquor was evaporated to give 115.5 g of a green foam. Dissolved this material in 500 mL of 1:1 dichloromethane/hexanes then loaded onto a 3 kg silica gel column (column volume = 4800 mL, flow rate = 900 mL/min). Ran a gradient of 2% to 9% EtOAc in hexanes over 43 minutes (8 column volumes), then ran at 9% EtOAc until the product finished eluting giving 77.8 g of impure product. This material was co-evaporated with methanol (~500 mL) then diluted with methanol (200 mL) to give 234.5 g of a methanolic solution, which was halved and each half was purified by reverse phase chromatography (3.8 kg C₁₈ column, column volume = 3300 mL, flow rate = 375 mL/min, loaded as solution in methanol). Ran the column at 55% acetonitrile for ~5 minutes (0.5 column volumes), then at a gradient of 55% to 100% acetonitrile in water over ~170 minutes (19-20 column volumes), then held at 100% acetonitrile until the product and impurities finished eluting. Clean product fractions from both columns were combined and concentrated by rotary evaporation then transferred with ethanol into 5 L flask, evaporated and carefully dried (becomes a foam) to give as a mixture of olefin isomers, *tert*-butyl *N*-[(6*R*,12*R*)-6-benzyloxy-12-methyl-6,15-bis(trifluoromethyl)-13,19-dioxa-3,4,18-triazatricyclo[12.3.1.12,5]nonadeca-1(18),2,4,9,14,16-hexaen-17-yl]-*N*-*tert-*butoxycarbonyl-carbamate (*E*/*Z* mixture) (55.5 g, 53%). ESI-MS *m*/*z* calc. 728.26447, found 729.0 (M+1)⁺; Retention time: 3.82 minutes. Final purity was determined by reversed phase UPLC using an Acquity UPLC BEH C₁₈ column (50 × 2.1 mm, 1.7 µm particle) made by Waters (pn: 186002350), and a dual gradient run from 1 - 99% mobile phase B over 4.5 minutes. Mobile phase A = water (0.05 % CF₃CO₂H). Mobile phase B = acetonitrile (0.035 % CF₃CO₂H). Flow rate = 1.2 mL/min, injection volume = 1.5 µL, and column temperature = 60 °C.

### Step 3: tert-Butyl N-[(6R,12R)-6-benzyloxy-12-methyl-6,15-bis(trifluoromethyl)-13,19-dioxa-3,4,18-triazatricyclo[12.3.1.12,5]nonadeca-1(18),2,4,14,16-pentaen-17-y1]-N-tert-butoxycarbonyl-carbamate

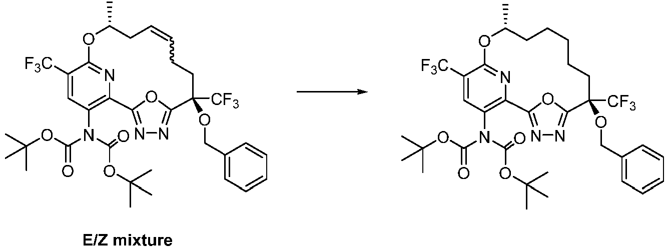

*tert*-Butyl *N*-[(6*R*,12*R*)-6-benzyloxy-12-methyl-6,15-bis(trifluoromethyl)-13,19-dioxa-3,4,18-triazatricyclo[12.3.1.12,5]nonadeca-1(18),2,4,9,14,16-hexaen-17-yl]-*N*-*tert*-butoxycarbonyl-carbamate (*E*/*Z* mixture) (11.7 g, 16.06 mmol) was dissolved in stirring ethanol (230 mL) and cycled the flask 3 times vacuum/nitrogen and treated with 10% Pd/C (50% water wet, 2.2 g of 5 % w/w, 1.034 mmol). The mixture was cycled 3 times between vacuum/nitrogen and 3 times between vacuum/hydrogen. The mixture was then stirred strongly under hydrogen (balloon) for 7.5 h. The catalyst was removed by filtration, replaced with fresh 10% Pd/C (50% water wet, 2.2 g of 5% w/w, 1.034 mmol) and stirred vigorously under hydrogen (balloon) overnight. Then, the catalyst was removed again by filtration, the filtrate evaporated and the residue (11.3 g, 1 g set aside) was dissolved in ethanol (230 mL) charged with fresh 10% Pd/C (50% water wet, 2.2 g of 5 % w/w, 1.034 mmol) and stirred vigorously under hydrogen (balloon) for 6 h, recharged again with fresh 10% Pd/C (50% water wet, 2.2 g of 5 % w/w, 1.034 mmol) and stirred vigorously under hydrogen (balloon) overnight. The catalyst was removed by filtration and the filtrate was evaporated (10 g of residue obtained). This crude material (10 g + 1 g set aside above) was purified by silica gel chromatography (330 g column, liquid load in dichloromethane) with a linear gradient of 0% to 15% ethyl acetate in hexane until the product eluted followed by 15% to 100% ethyl acetate in hexane to giving, as a colorless foam, *tert*-butyl *N*-[(6*R*,12*R*)-6-benzyloxy-12-methyl-6,15-bis(trifluoromethyl)-13,19-dioxa-3,4,18-triazatricyclo[12.3.1.12,5]nonadeca-1(18),2,4, 14, 16-pentaen-17-yl]-*N-tert*-butoxycarbonyl-carbamate (9.1 g, 78%). ESI-MS *m*/*z* calc. 730.2801, found 731.0 (M+1)⁺ ; Retention time: 3.89 minutes. Final purity was determined by reversed phase UPLC using an Acquity UPLC BEH C₁₈ column (50 × 2.1 mm, 1.7 µm particle) made by Waters (pn: 186002350), and a dual gradient run from 1 - 99% mobile phase B over 4.5 minutes. Mobile phase A = water (0.05 % CF₃CO₂H). Mobile phase B = acetonitrile (0.035 % CF₃CO₂H). Flow rate = 1.2 mL/min, injection volume = 1.5 µL, and column temperature = 60 °C.

### Step 4: (6R,12R)-17-Amino-12-methyl-6,15-bis(trifluoromethyl)-13,19-dioxa-3,4,18-triazatricyclo[12.3.1.12,5]nonadeca-1(18),2,4,14,16-pentaen-6-ol, Compound I

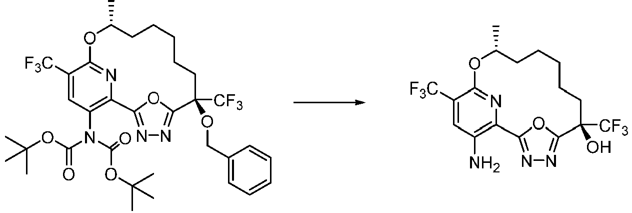

*tert*-Butyl *N*-[(6*R*,12*R*)-6-benzyloxy-12-methyl-6,15-bis(trifluoromethyl)-13,19-dioxa-3,4,18-triazatricyclo[12.3.1.12,5]nonadeca-1(18),2,4,14,16-pentaen-17-yl]-*N*-*tert*-butoxycarbonyl-carbamate (8.6 g, 11.77 mmol) was dissolved in ethanol (172 mL) then the flask was cycled 3 times between vacuum/nitrogen. Treated the mixture with 10% Pd/C (50% water wet, 1.8 g of 5 % w/w, 0.8457 mmol) then cycled 3 times between vacuum/nitrogen and 3 times between vacuum/hydrogen and then stirred vigorously under hydrogen (balloon) at room temperature for 18 h. The mixture was cycled 3 times between vacuum/nitrogen, filtered over Celite washing with ethanol and then the filtrate was evaporated to give 7.3 g of *tert*-butyl *N*-*tert*-butoxycarbonyl-*N*-[(6*R*,12*R*)-6-hydroxy-12-methyl-6,15-bis(trifluoromethyl)-13,19-dioxa-3,4,18-triazatricyclo[12.3.1.12,5]nonadeca-1(18),2,4,14,16-pentaen-17-yl]carbamate an offwhite solid. This material was dissolved in dichloromethane (69 mL), cooled in an ice bath under nitrogen and slowly treated with TFA (23 mL, 298.5 mmol). The solution was stirred in the ice bath for 5 min and then at room temperature for 1 h. The paleyellow solution was diluted with heptane (~100 mL) and evaporated to give a yellow solid mass. The residue was diluted again with heptane (~100 - 200 mL) and dichloromethane was added under warming until a yellow solution was obtained. Most of the dichloromethane was removed by rotary evaporation (35 °C water bath, 100 mbar pressure) to give a fine yellow suspension. The suspension was swirled for ~1 h at room temperature, filtered washing the solid with dry ice chilled heptane and then dried over 3 days under vacuum with a nitrogen leak at 50 °C to give as a pale yellow solid, (6*R*,12*R*)-17-amino-12-methyl-6,15-bis(trifluoromethyl)-13,19-dioxa-3,4,18-triazatricyclo[12.3.1.12,5]nonadeca-1(18),2,4,14,16-pentaen-6-ol (4.68 g, 90%). ¹H NMR (400 MHz, DMSO-d₆) δ 7.77 (s, 1H), 7.55 (s, 1H), 6.34 (s, 2H), 4.90 - 4.70 (m, 1H), 2.47 (dd, *J =* 7.8, 5.5 Hz, 1H), 2.29 (t*, J =* 11.2 Hz, 1H), 2.11 (ddd, *J* = 14.4, 8.7, 6.1 Hz, 1H), 1.73 (dt, *J =* 12.7, 7.6 Hz, 2H), 1.59 - 1.38 (m, 4H), 1.35 (d, *J =* 6.3 Hz, 3H), 1.18 (ddt, *J =* 12.4, 9.6, 6.2 Hz, 1H) ppm. ¹H NMR (400 MHz, Chloroform-d) δ 7.42 (d, *J = 0.8* Hz, 1H), 5.20 (s, 2H), 4.75 (dtt, *J =* 12.6, 6.3, 3.2 Hz, 1H), 3.98 (s, 1H), 2.68 (dtd, *J =* 12.9, 7.6, 2.3 Hz, 1H), 2.38 - 2.18 (m, 2H), 2.03 (d, *J =* 7.9 Hz, 1H), 1.75 - 1.46 (m, 5H), 1.41 (d, *J =* 6.3 Hz, 3H), 1.35 - 1.27 (m, 1H) ppm. ¹⁹F NMR (376 MHz, Chloroform-d) δ -63.95, -77.34 ppm. ESI-MS *m*/*z* calc. 440.1283, found 441.0 (M+1)⁺; Retention time: 2.87 minutes. Final purity was determined by reversed phase UPLC using an Acquity UPLC BEH C₁₈ column (50 × 2.1 mm, 1.7 µm particle) made by Waters (pn: 186002350), and a dual gradient run from 1 - 99% mobile phase B over 4.5 minutes. Mobile phase A = water (0.05 % CF₃CO₂H). Mobile phase B = acetonitrile (0.035 % CF₃CO₂H). Flow rate = 1.2 mL/min, injection volume = 1.5 µL, and column temperature = 60 °C.

### Example 2: Bioactivity Assay

### Ussing Chamber Assay of CFTR-mediated short-circuit currents

Ussing chamber experiments were performed using human bronchial epithelial (HBE) cells derived from CF subjects heterozygous for F508del and a minimal function CFTR mutation (F508del/MF-HBE) and cultured as previously described (Neuberger T., Burton B., Clark H., Van Goor F. Methods Mol Biol 2011:741:39-54). After four days the apical media was removed, and the cells were grown at an air liquid interface for >14 days prior to use. This resulted in a monolayer of fully differentiated columnar cells that were ciliated, features that are characteristic of human bronchial airway epithelia.

To isolate the CFTR-mediated short-circuit (I_{SC}) current, F508del/MF-HBE grown on Costar^{®} Snapwell^{™} cell culture inserts were mounted in an Ussing chamber and the transepithelial I_{SC} was measured under voltage-clamp recording conditions (V_{hold}= 0 mV) at 37°C. The basolateral solution contained (in mM) 145 NaCl, 0.83 K₂HPO₄, 3.3 KH₂PO₄, 1.2 MgCl₂, 1.2 CaCl₂, 10 Glucose, 10 HEPES (pH adjusted to 7.4 with NaOH) and the apical solution contained (in mM) 145 NaGluconate, 1.2 MgCl₂, 1.2 CaCl₂, 10 glucose, 10 HEPES (pH adjusted to 7.4 with NaOH) and 30 µM amiloride to block the epithelial sodium channel. Forskolin (20 µM) was added to the apical surface to activate CFTR, followed by apical addition of a CFTR inhibitor cocktail consisting of BPO, GlyH-101 and CFTR inhibitor 172 (each at 20 µM final assay concentration) to specifically isolate CFTR currents. The CFTR-mediated I_{SC} (µA/cm²) for each condition was determined from the peak forskolin response to the steady-state current following inhibition.

### Identification of Potentiator Compounds

The activity of the CFTR potentiator compounds on the CFTR-mediated I_{SC} was determined in Ussing chamber studies as described above. The F508del/MF-HBE cell cultures were incubated with the potentiator compounds at a range of concentrations in combination with 10 µM (14*S*)-8-[3-(2-{ dispiro[2.0.2.1]heptan-7-yl}ethoxy)-1H-pyrazol-1-yl]-12,12-dimethyl-2λ⁶-thia-3,9,11,18,23-pentaazatetracyclo [17.3.1.111,14.05,10]tetracosa-1(22),5,7,9,19(23),20-hexaene-2,2,4-trione for 18 - 24 hours at 37°C and in the presence of 20% human serum. The concentration of potentiator compounds and (14*S*)-8-[3-(2-{dispiro[2.0.2.1]heptan-7-yl}ethoxy)-1H-pyrazol-1-yl]-12,12-dimethyl-2λ⁶-thia-3,9,11,18,23-pentaazatetracyclo [17.3.1.111,14.05,10]tetracosa-1(22),5,7,9,19(23),20-hexaene-2,2,4-trione used during the 18-24 hours incubations was kept constant throughout the Ussing chamber measurement of the CFTR-mediated I_{SC} to ensure compounds were present throughout the entire experiment. The efficacy and potency of the putative F508del potentiators was compared to that of the known Vertex potentiator ivacaftor (*N*-[2,4-bis(1,1-dimethylethyl)-5-hydroxyphenyl]-1,4-dihydro-4-oxoquinoline-3-carboxamide or *N*-(5-hydroxy-2,4-di-*tert*-butyl-phenyl)-4-oxo-1*H*-quinoline-3-carboxamide).

**Table 3** presents CFTR-modulating activity for Compound **I** using the assay described in this example (EC₅₀: +++ is < 500 nM).

**Table 3: Assay Data for Compound I**

| **Molecule** | **EC₅₀** |
|---|---|
| Compound **I** | +++ |

### Example 3

### Study Rationale

In a first-in-human (FIH) clinical study of Compound **I,** designed to evaluate the safety, tolerability, and pharmacokinetics (PK) of Compound **I** in healthy subjects, an assessment of drug-drug interactions (DDIs) between Compound **I** and itraconazole and midazolam was conducted.

### Study Design

This first-in-human (FIH) study of Compound **I** included three parts (Parts A, B, and C).

Approximately 154 healthy adult subjects were planned to be enrolled in planned cohorts. Approximately 76 subjects were planned to be enrolled in Part A, approximately 48 subjects in Part B, and approximately 30 subjects in Part C. There was no pre-specified ratio of males to females, but reasonable effort was made to enroll females of non-childbearing potential in all dosing cohorts. The final number of subjects in each analysis set is provided in **Table 4.**

**Table 4: Analysis Population**

| | **Part A** | **Part B** | **Part C** | **Overall** |
|---|---|---|---|---|
| **All Subjects Set** | 101 | 47 | 30 | 178 |
| **Safety Set** | 101 | 47 | 30 | 178 |

| | | | | |
|---|---|---|---|---|
| Notes: All Subjects Set: all subjects in the study who were either randomized (for Part A Cohorts except A9 and Part B cohorts) or enrolled (for Cohort A9, C1, C2; defined as subject having data in the clinical database), or received at least 1 dose of study drug; Safety Set: all subjects who received at least 1 dose of study drug. Part A included Cohorts A1 through A8, A9, and A10. Part B included Cohorts B1 through B6. Part C included Cohorts C1 and C2. | | | | |

### Parts A and B:

Six dose escalation cohorts each were planned for Parts A and B. Additional cohorts in Part A were enrolled based on data from previous cohorts. In each cohort, approximately eight subjects were randomized 3:1 to receive Compound **I** or placebo as a suspension, under fasted or fed conditions. Eight subjects were enrolled in food-effect Cohort A9 to receive open-label Compound **I;** subsequent cohorts were dosed under fed conditions. Twenty-eight subjects were enrolled in relative bioavailability and food-effect Cohort A10 to receive open-label Compound **I in** Cohort A10 . The decision to initiate successive cohorts and dose selection was based on safety and tolerability data from the preceding dose group(s), as well as available PK data from the preceding cohorts.

### Part A:

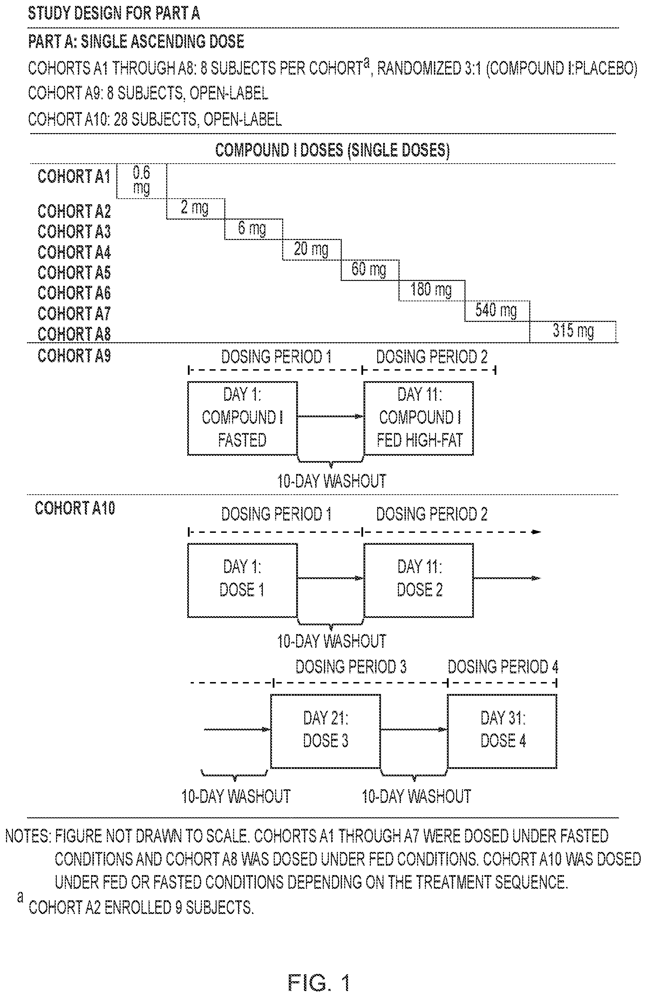

Part A (except Cohorts A9 and A10) was a randomized, double-blind, placebo-controlled study evaluating single ascending doses of Compound **I** administered to healthy subjects (**FIG. 1**). **FIG. 1** provides the doses that were studied. The starting dose (Cohort A1) was 0.6 mg. The doses for Cohort A2, A3, A4, A5, A6, A7, and A8 were 2 mg, 6 mg, 20 mg, 60 mg, 180 mg, 540 mg, and 315 mg, respectively. Subject dosing was staggered in Part A (all cohorts excluding Cohorts A9 and A10) so that two sentinel subjects were dosed (one with Compound **I** and one with placebo) at least 48 hours before the remaining six subjects in each cohort. The remaining subjects in the cohort were dosed after safety data from the sentinel subjects was reviewed. Dose escalation occurred at increments of a maximum of half-log (3.3-fold) initially and the incremental increase occurred at no greater than two-fold as exposures approached PK stopping criteria. Doses were repeated or modified based on emerging data.

Food effect was evaluated in open-label, single-sequence Cohort A9 to evaluate the effect of food on the PK of Compound **I** based on emerging PK data during dose escalation.

Cohort A10 was a randomized, open label, single dose, 4-period, 4-sequence, crossover study to evaluate the relative bioavailability of Suspension 2 of Compound **I** compared to Suspension 1 of Compound **I** and the effect of food (high-fat and low-fat) on the PK of the Suspension 2 of Compound **I** to inform formulation development. Suspension 1 and Suspension 2 had different compositions. Cohort A10 was the only cohort in which Suspension 2 was administered.

Subjects in Cohort A10 were equally randomized (1:1:1:1) on Day 1 to one of 4 treatment sequences using a balanced Latin Square design (**Table 5**). There were 4 treatments:
Treatment A: Compound **I** Suspension 1, fasted
Treatment B: Compound **I** Suspension 2, fasted
Treatment C: Compound **I** Suspension 2, fed high-fat
Treatment D: Compound **I** Suspension 2, fed low-fat

**Table 5: Dosing Periods and Treatment Sequences for Cohort A10**

| **Treatment Sequence (n)** | **Dosing Period 1** | **Dosing Period 2** | **Dosing Period 3** | **Dosing Period 4** |
|---|---|---|---|---|
| 1 (n = 7) | A | B | C | D |
| 2 (n = 7) | B | C | D | A |
| 3 (n = 7) | C | D | A | B |
| 4 (n = 7) | D | A | B | C |

| | | | | |
|---|---|---|---|---|
| n: number of subjects Notes: Treatment A: Compound **I** Suspension 1, fasted; Treatment B: Compound **I** Suspension 2, fasted; Treatment C: Compound **I** Suspension 2, fed high-fat; Treatment D: Compound **I** Suspension 2, fed low-fat. | | | | |

### Part B:

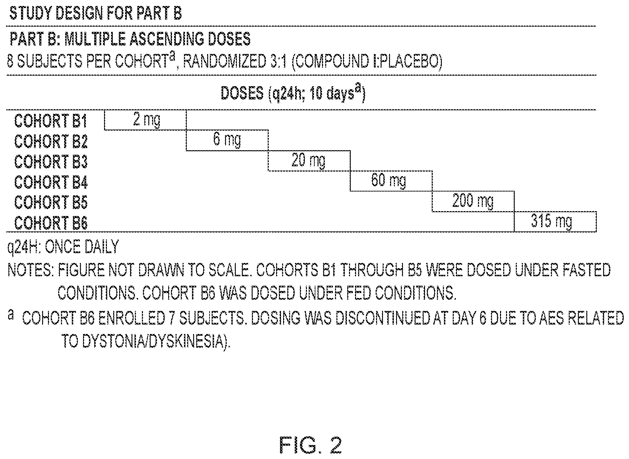

Part B was a randomized, double-blind, and placebo-controlled study evaluating multiple ascending doses of Compound **I** administered to healthy subjects (**FIG. 2**). **FIG. 2** provides the doses that were studied. Part B was initiated while Part A was ongoing after review of safety, tolerability, and PK data. The total daily doses for Cohort B1, B2, B3, B4, B5, and B6 were 2 mg, 6 mg, 20 mg, 60 mg, 200 mg, and 315 mg, respectively. In Part B, sentinel dosing occurred in each cohort until the full range of anticipated exposures had been explored in Part A. The first two subjects were dosed (one with Compound **I** and one with placebo) at least five days before the remaining subjects in the cohort. The remaining subjects in the cohort were dosed after available safety data from the sentinel subjects were reviewed. Dose escalation occurred at increments of a maximum of half-log (3.3-fold) initially and the incremental increase occurred at no greater than two-fold as exposures approached PK stopping criteria. Subjects in Cohorts B1 to B5 were dosed for ten days. Subjects in Cohort B6 were dosed for 6 days.

### Part C:

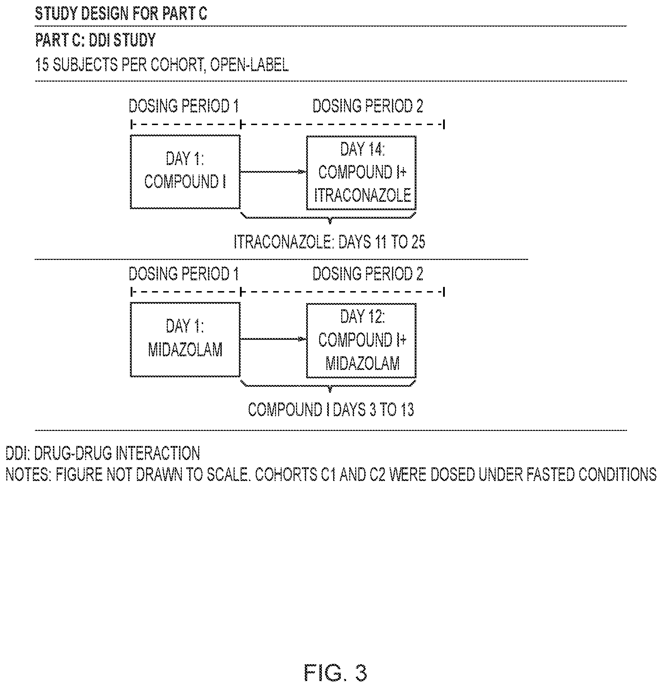

Part C was an open-label, single-sequence, DDI study (**FIG. 3**). Fifteen subjects were enrolled in each cohort. Cohort C1 evaluated the effect of itraconazole on the PK of single doses of Compound **I.** Cohort C2 evaluated the PK of midazolam in the absence and presence of Compound **I.**

### Conclusions

Compound **I** was generally safe and well-tolerated across all cohorts.

## Claims

1. Compound **I**: 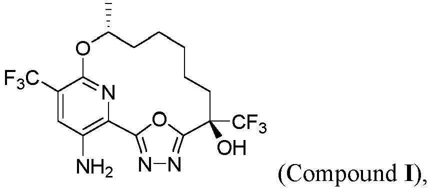 or a deuterated derivative or pharmaceutically acceptable salt thereof, for use in a method of treating cystic fibrosis in a patient comprising administering about 0.1 mg to about 50 mg of Compound **I** or an equivalent amount of a deuterated derivative or pharmaceutically acceptable salt thereof daily.

2. The compound for use according to claim 1, wherein the use comprises administering about 0.25 mg to about 50 mg of Compound **I,** or an equivalent amount of a deuterated derivative or a pharmaceutically acceptable salt thereof daily; for example,
about 0.25 mg to about 25 mg of Compound **I** or an equivalent amount of a deuterated derivative or a pharmaceutically acceptable salt thereof daily; for example,
about 1 mg to about 15 mg of Compound **I** or an equivalent amount of a deuterated derivative or a pharmaceutically acceptable salt thereof daily; for example ,
about 1.5 mg to about 14 mg of Compound **I** or an equivalent amount of a deuterated derivative or a pharmaceutically acceptable salt thereof daily; for example,
about 4 mg to about 13 mg of Compound **I** or an equivalent amount of a deuterated derivative or a pharmaceutically acceptable salt thereof daily; for example,
about 8 mg to about 13 mg of Compound **I** or an equivalent amount of a deuterated derivative or a pharmaceutically acceptable salt thereof daily.

3. The compound for use according to claim 1, wherein:
(a) the use comprises administering about 10 mg to about 40 mg of Compound **I** or an equivalent amount of a deuterated derivative or a pharmaceutically acceptable salt thereof daily; or
(b) about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg of Compound **I** or an equivalent amount of a deuterated derivative or a pharmaceutically acceptable salt thereof is administered daily.

4. The compound for use according to claim 1, wherein the patient is heterozygous and has one F508del mutation.

5. The compound for use according to claim 4, wherein the use comprises administering:
about 0.25 mg to about 50 mg of Compound **I** or an equivalent amount of a deuterated derivative or a pharmaceutically acceptable salt thereof daily; for example,
about 0.25 mg to about 25 mg of Compound **I** or an equivalent amount of a deuterated derivative or a pharmaceutically acceptable salt thereof daily; for example,
about 1 mg to about 15 mg of Compound **I** or an equivalent amount of a deuterated derivative or a pharmaceutically acceptable salt thereof daily; for example,
about 1.5 mg to about 14 mg of Compound **I** or an equivalent amount of a deuterated derivative or a pharmaceutically acceptable salt thereof daily; for example,
about 4 mg to about 13 mg of Compound **I** or an equivalent amount of a deuterated derivative or a pharmaceutically acceptable salt thereof daily; for example,
about 8 mg to about 13 mg of Compound **I** or an equivalent amount of a deuterated derivative or a pharmaceutically acceptable salt thereof daily.

6. The compound for use according to claim 4, wherein:
(a) the use comprises administering about 10 mg to about 40 mg of Compound **I** or an equivalent amount of a deuterated derivative or a pharmaceutically acceptable salt thereof daily; or
(b) about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg of Compound **I** or an equivalent amount of a deuterated derivative or a pharmaceutically acceptable salt thereof is administered daily.

7. The compound for use according to any one of claims 1-6, wherein Compound **I** or a deuterated derivative or a pharmaceutically acceptable salt thereof is administered:
(a) as a single dose, once daily; and/or
(b) in two doses daily.

8. The compound for use according to any one of claims 1-7, further comprising administering one or more additional therapeutic agent(s).

9. The compound for use according to claim 8, wherein the one or more additional therapeutic agent(s) comprise(s) a compound selected from:
(a) (*R*)-1-(2,2-difluorobenzo[d][1,3]dioxol-5-yl)-*N*-(1-(2,3-dihydroxypropyl)-6-fluoro-2-(1-hydroxy-2-methylpropan-2-yl)-1*H*-indol-5-yl)cyclopropanecarboxamide (Compound **II**): 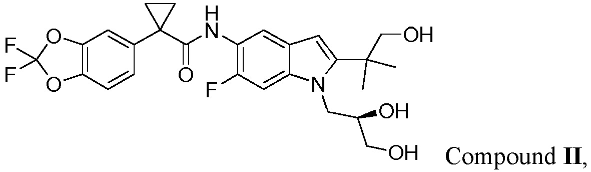
(b) 3-(6-(1-(2,2-difluorobenzo[d][1,3]dioxol-5-yl)cyclopropanecarboxamido)-3-methylpyridin-2-yl)benzoic acid (Compound **IV**): 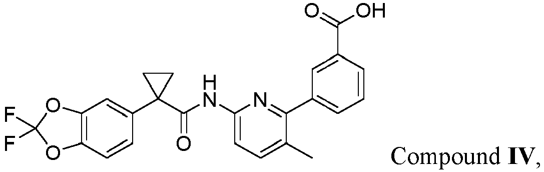
(c) *N*-(1,3-dimethylpyrazol-4-yl)sulfonyl-6-[3-(3,3,3-trifluoro-2,2-dimethyl-propoxy)pyrazol-1-yl]-2-[(4*S*)-2,2,4-trimethylpyrrolidin-1-yl]pyridine-3-carboxamide (Compound V): 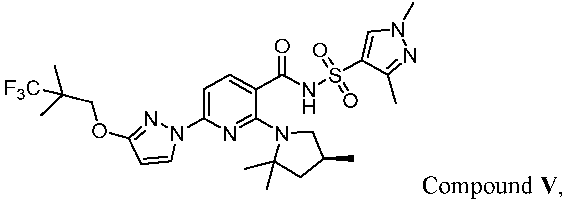
(d) *N*-(benzenesulfonyl)-6-[3-[2-[1-(trifluoromethyl) cyclopropyl]ethoxy]pyrazol-1-yl]-2-[(4*S*)-2,2,4-trimethylpyrrolidin-1-yl]pyridine-3-carboxamide (Compound VI): 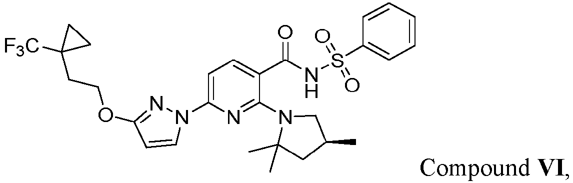
(e) (14*S*)-8-[3-(2-{dispiro[2.0.2.1]heptan-7-yl}ethoxy)-1*H*-pyrazol-1-yl]-12,12-dimethyl-2λ⁶-thia-3,9,11,18,23-pentaazatetracyclo [17.3.1.111,14.05,10]tetracosa-1(22),5,7,9,19(23),20-hexaene-2,2,4-trione (Compound **VII**): 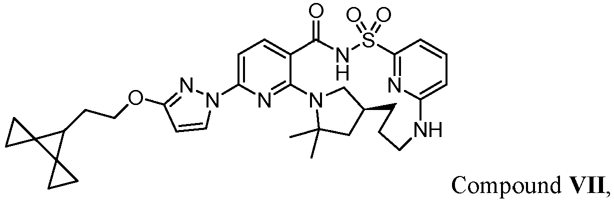
(f) (11*R*)-6-(2,6-dimethylphenyl)-11-(2-methylpropyl)-12-{spiro[2.3]hexan-5-yl}-9-oxa-2λ⁶-thia-3,5,12,19-tetraazatricyclo[12.3.1.14,8]nonadeca-1(17),4(19),5,7,14(18),15-hexaene-2,2,13-trione (Compound VIII): 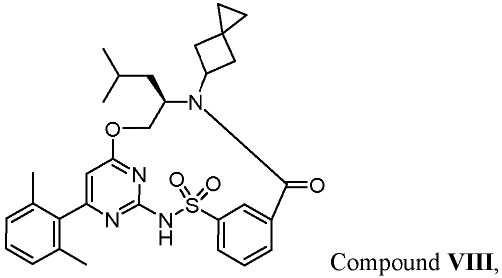
(g) *N*-(benzenesulfonyl)-6-(3-fluoro-5-isobutoxy-phenyl)-2-[(4*S*)-2,2,4-trimethylpyrrolidin-1-yl]pyridine-3-carboxamide (Compound IX): 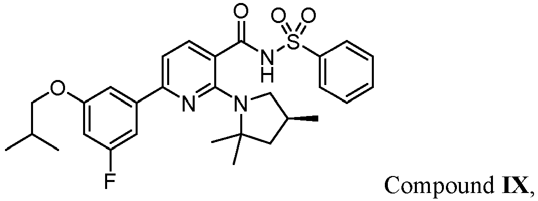
(h) *N*-[(6-amino-2-pyridyl)sulfonyl]-6-(3-fluoro-5-isobutoxy-phenyl)-2-[(4*S*)-2,2,4-trimethylpyrrolidin-1-yl]pyridine-3-carboxamide (Compound X): 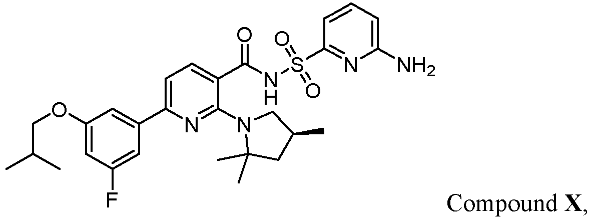 and deuterated derivatives and pharmaceutically acceptable salts thereof.

10. The compound for use according to claim 8 or claim 9, wherein the one or more additional therapeutic agent(s) comprise(s) a compound selected from:
(a) *N*-(5-hydroxy-2,4-di-*tert*-butyl-phenyl)-4-oxo-1*H*-quinoline-3-carboxamide (Compound **III**): 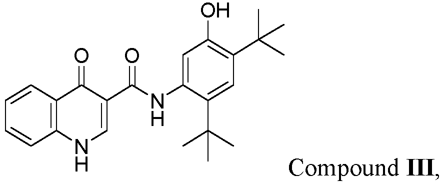
(b) *N*-(2-(*tert*-butyl)-5-hydroxy-4-(2-(methyl-d3)propan-2-yl-1,1,1,3,3,3-d6)phenyl)-4-oxo-1,4-dihydroquinoline-3-carboxamide (Compound **III-d**): 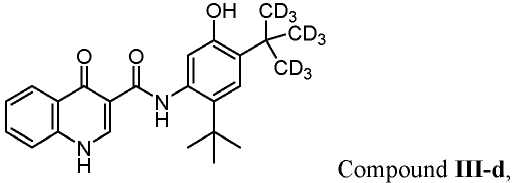 and deuterated derivatives and pharmaceutically acceptable salts thereof.

11. A pharmaceutical composition comprising about 0.1 mg to about 50 mg of Compound **I**: 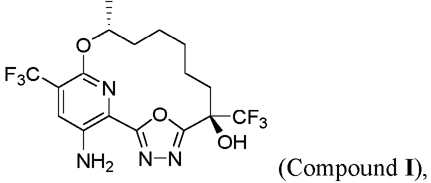 or an equivalent amount of a deuterated derivative or a pharmaceutically acceptable salt thereof.

12. The pharmaceutical composition according to claim 11, wherein the composition comprises:
about 0.25 mg to about 50 mg of Compound **I** or an equivalent amount of a deuterated derivative or a pharmaceutically acceptable salt thereof; for example,
about 0.25 mg to about 25 mg of Compound **I** or an equivalent amount of a deuterated derivative or a pharmaceutically acceptable salt thereof; for example,
about 1 mg to about 15 mg of Compound **I** or an equivalent amount of a deuterated derivative or a pharmaceutically acceptable salt thereof; for example,
about 1.5 mg to about 14 mg of Compound **I** or an equivalent amount of a deuterated derivative or a pharmaceutically acceptable salt thereof; for example,
about 4 mg to about 13 mg of Compound **I** or an equivalent amount of a deuterated derivative or a pharmaceutically acceptable salt thereof; for example,
about 8 mg to about 13 mg of Compound **I** or an equivalent amount of a deuterated derivative or a pharmaceutically acceptable salt thereof.

13. The pharmaceutical composition according to claim 11, wherein the composition comprises:
(a) about 10 mg to about 40 mg of Compound **I** or an equivalent amount of a deuterated derivative or a pharmaceutically acceptable salt thereof; or
(b) about 0.1 mg, about 0.25 mg, about 1 mg, about 1.5 mg, about 2.5 mg, about 4 mg, about 4.5 mg, about 5 mg, about 5.5 mg, about 6 mg, about 6.5 mg, about 7 mg, about 7.5 mg, about 8 mg, about 8.5 mg, about 9, about 9.5 mg, about 10 mg, about 10.5 mg, about 11 mg, about 11.5 mg, about 12 mg, about 12.5 mg, about 13 mg, about 14 mg, about 15 mg, about 17.5 mg, about 20 mg, about 22.5 mg, about 25 mg, about 27.5 mg, about 30 mg, about 32.5 mg, about 35 mg, about 37.5 mg, about 40 mg, about 42.5 mg, about 45 mg, about 47.5 mg, or about 50 mg of Compound **I** or an equivalent amount of a deuterated derivative or a pharmaceutically acceptable salt thereof.

14. The pharmaceutical composition according to any one of claims 11-13, wherein the composition further comprises one or more additional therapeutic agent(s).

15. The pharmaceutical composition according to claim 14, wherein the one or more additional therapeutic agent(s) comprise(s) a compound with CFTR-modulating activity or a deuterated derivative or a pharmaceutically acceptable salt thereof.

## Patentansprüche

1. Verbindung I: 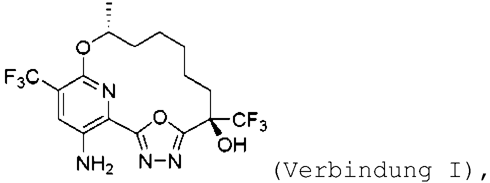 oder ein deuteriertes Derivat oder pharmazeutisch annehmbares Salz davon zur Verwendung bei einem Verfahren zur Behandlung von zystischer Fibrose bei einem Patienten, umfassend Verabreichen von etwa 0,1 mg bis etwa 50 mg Verbindung I oder einer äquivalenten Menge eines deuterierten Derivats oder pharmazeutisch annehmbaren Salzes davon täglich.

2. Verbindung zur Verwendung nach Anspruch 1, wobei die Verwendung Verabreichen von etwa 0,25 mg bis etwa 50 mg Verbindung I oder einer äquivalenten Menge eines deuterierten Derivats oder eines pharmazeutisch annehmbaren Salzes davon täglich umfasst; beispielsweise etwa 0,25 mg bis etwa 25 mg Verbindung I oder eine äquivalente Menge eines deuterierten Derivats oder eines pharmazeutisch annehmbaren Salzes davon täglich; beispielsweise
etwa 1 mg bis etwa 15 mg Verbindung I oder eine äquivalente Menge eines deuterierten Derivats oder eines pharmazeutisch annehmbaren Salzes davon täglich; beispielsweise
etwa 1,5 mg bis etwa 14 mg Verbindung I oder eine äquivalente Menge eines deuterierten Derivats oder eines pharmazeutisch annehmbaren Salzes davon täglich; beispielsweise
etwa 4 mg bis etwa 13 mg Verbindung I oder eine äquivalente Menge eines deuterierten Derivats oder eines pharmazeutisch annehmbaren Salzes davon täglich; beispielsweise
etwa 8 mg bis etwa 13 mg Verbindung I oder eine äquivalente Menge eines deuterierten Derivats oder eines pharmazeutisch annehmbaren Salzes davon täglich.

3. Verbindung zur Verwendung nach Anspruch 1, wobei:
(a) die Verwendung Verabreichen von etwa 10 mg bis etwa 40 mg Verbindung I oder einer äquivalenten Menge eines deuterierten Derivats oder eines pharmazeutisch annehmbaren Salzes davon täglich umfasst; oder
(b) etwa 0,1 mg, etwa 0,25 mg, etwa 1 mg, etwa 1,5 mg, etwa 2,5 mg, etwa 4 mg, etwa 4,5 mg, etwa 5 mg, etwa 5,5 mg, etwa 6 mg, etwa 6,5 mg, etwa 7 mg, etwa 7,5 mg, etwa 8 mg, etwa 8,5 mg, etwa 9, etwa 9,5 mg, etwa 10 mg, etwa 10,5 mg, etwa 11 mg, etwa 11,5 mg, etwa 12 mg, etwa 12,5 mg, etwa 13 mg, etwa 14 mg, etwa 15 mg, etwa 17,5 mg, etwa 20 mg, etwa 22,5 mg, etwa 25 mg, etwa 27,5 mg, etwa 30 mg, etwa 32,5 mg, etwa 35 mg, etwa 37,5 mg, etwa 40 mg, etwa 42,5 mg, etwa 45 mg, etwa 47,5 mg oder etwa 50 mg Verbindung I oder eine äquivalente Menge eines deuterierten Derivats oder eines pharmazeutisch annehmbaren Salzes davon täglich verabreicht werden.

4. Verbindung zur Verwendung nach Anspruch 1, wobei der Patient heterozygot ist und eine F508del-Mutation aufweist.

5. Verbindung zur Verwendung nach Anspruch 4, wobei die Verwendung Verabreichen umfasst:
etwa 0,25 mg bis etwa 50 mg Verbindung I oder eine äquivalente Menge eines deuterierten Derivats oder eines pharmazeutisch annehmbaren Salzes davon täglich; beispielsweise
etwa 0,25 mg bis etwa 25 mg Verbindung I oder eine äquivalente Menge eines deuterierten Derivats oder eines pharmazeutisch annehmbaren Salzes davon täglich; beispielsweise
etwa 1 mg bis etwa 15 mg Verbindung I oder eine äquivalente Menge eines deuterierten Derivats oder eines pharmazeutisch annehmbaren Salzes davon täglich; beispielsweise
etwa 1,5 mg bis etwa 14 mg Verbindung I oder eine äquivalente Menge eines deuterierten Derivats oder eines pharmazeutisch annehmbaren Salzes davon täglich; beispielsweise
etwa 4 mg bis etwa 13 mg Verbindung I oder eine äquivalente Menge eines deuterierten Derivats oder eines pharmazeutisch annehmbaren Salzes davon täglich; beispielsweise
etwa 8 mg bis etwa 13 mg Verbindung I oder eine äquivalente Menge eines deuterierten Derivats oder eines pharmazeutisch annehmbaren Salzes davon täglich.

6. Verbindung zur Verwendung nach Anspruch 4, wobei:
(a) die Verwendung Verabreichen von etwa 10 mg bis etwa 40 mg Verbindung I oder einer äquivalenten Menge eines deuterierten Derivats oder eines pharmazeutisch annehmbaren Salzes davon täglich umfasst; oder
(b) etwa 0,1 mg, etwa 0,25 mg, etwa 1 mg, etwa 1,5 mg, etwa 2,5 mg, etwa 4 mg, etwa 4,5 mg, etwa 5 mg, etwa 5,5 mg, etwa 6 mg, etwa 6,5 mg, etwa 7 mg, etwa 7,5 mg, etwa 8 mg, etwa 8,5 mg, etwa 9, etwa 9,5 mg, etwa 10 mg, etwa 10,5 mg, etwa 11 mg, etwa 11,5 mg, etwa 12 mg, etwa 12,5 mg, etwa 13 mg, etwa 14 mg, etwa 15 mg, etwa 17,5 mg, etwa 20 mg, etwa 22,5 mg, etwa 25 mg, etwa 27,5 mg, etwa 30 mg, etwa 32,5 mg, etwa 35 mg, etwa 37,5 mg, etwa 40 mg, etwa 42,5 mg, etwa 45 mg, etwa 47,5 mg oder etwa 50 mg Verbindung I oder eine äquivalente Menge eines deuterierten Derivats oder eines pharmazeutisch annehmbaren Salzes davon täglich verabreicht werden.

7. Verbindung zur Verwendung nach einem der Ansprüche 1-6, wobei Verbindung I oder ein deuteriertes Derivat oder ein pharmazeutisch annehmbares Salz verabreicht wird:
(a) als eine Einzeldosis einmal täglich; und/oder
(b) in zwei Dosen täglich.

8. Verbindung zur Verwendung nach einem der Ansprüche 1-7, ferner umfassend Verabreichen eines oder mehrerer zusätzlicher therapeutischer Mittel.

9. Verbindung zur Verwendung nach Anspruch 8, wobei das eine oder die mehreren zusätzlichen therapeutischen Mittel eine Verbindung umfassen ausgewählt aus:
(a) (R) -1- (2, 2-Difluorbenzo [d] [1, 3] dioxol-5-yl) -N-(1-(2,3-dihydroxypropyl)-6-fluor-2-(1-hydroxy-2-methylpropan-2-yl)-1H-indol-5-yl)cyclopropancarboxamid (Verbindung II): 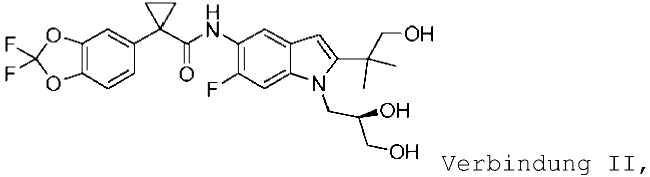
(b) 3-(6-(1-(2,2-Difluorbenzo[d][1,3]dioxol-5-yl)cyclopropancarboxamido)-3-methylpyridin-2-yl)benzoesäure (Verbindung IV): 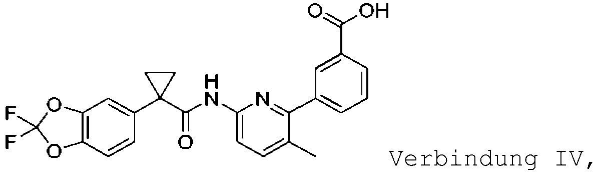
(c) N-(1,3-Dimethylpyrazol-4-yl)sulfonyl-6-[3-(3,3,3-trifluor-2,2-dimethylpropoxy)pyrazol-1-yl]-2-[(4S)-2,2,4-trimethylpyrrolidin-1-yl]pyridin-3-carboxamid (Verbindung V): 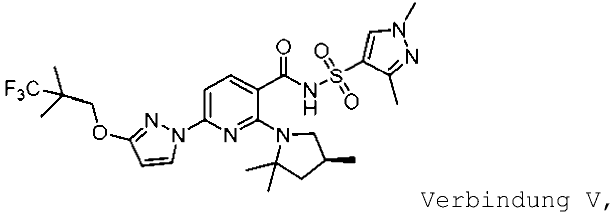
(d) N-(Benzolsulfonyl)-6-[3-[2-[1-(trifluormethyl)cyclopropyl]ethoxy]pyrazol-1-yl]-2-[(4S)-2,2,4-trimethylpyrrolidin-1-yl]pyridin-3-carboxamid (Verbindung VI): 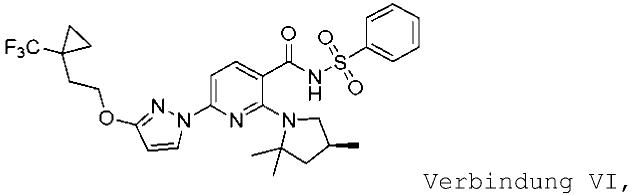
(e) (14S)-8-[3-(2-{Dispiro[2.0.2.1]heptan-7-yl}ethoxy)-1H-pyrazol-1-yl]-12,12-dimethyl-2λ⁶-thia-3,9,11,18,23-pentaazatetracyclo[17.3.1.111,14.05,10]tetracosa-1(22),5,7,9,19(23),20-hexaen-2,2,4-trion (Verbindung VII): 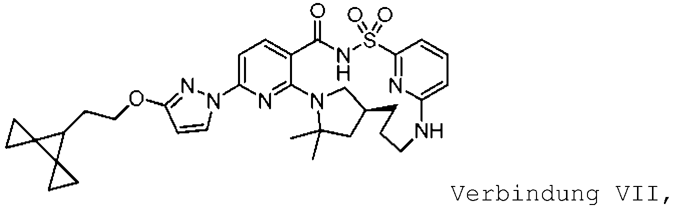
(f) (11R)-6-(2,6-Dimethylphenyl)-11-(2-methylpropyl)-12-{spiro[2.3]hexan-5-yl}-9-oxa-2λ⁶-thia-3,5,12,19-tetraazatricyclo[12.3.1.14,8]nonadeca-1(17),4(19),5,7,14(18),15-hexaen-2,2,13-trion (Verbindung VIII): 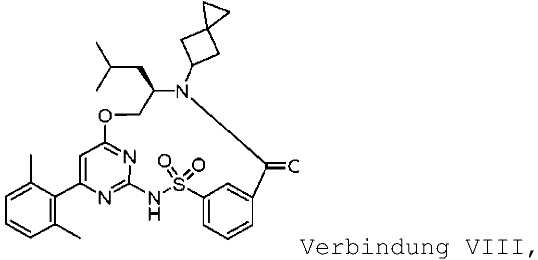
(g) N-(Benzolsulfonyl)-6-(3-fluor-5-isobutoxyphenyl)-2-[(4S)-2,2,4-trimethylpyrrolidin-1-yl]pyridin-3-carboxamid (Verbindung IX): 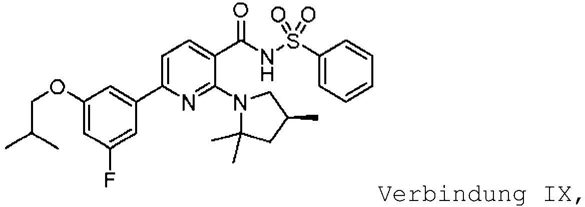
(h) N-[(6-Amino-2-pyridyl)sulfonyl]-6-(3-fluor-5-isobutoxyphenyl)-2-[(4S)-2,2,4-trimethylpyrrolidin-1-yl]pyridin-3-carboxamid (Verbindung X): 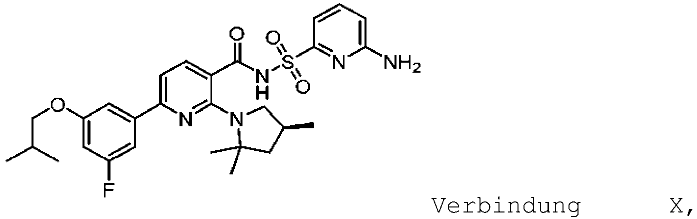 und deuterierte Derivate und pharmazeutisch annehmbare Salze davon.

10. Verbindung zur Verwendung nach Anspruch 8 oder Anspruch 9, wobei das eine oder die mehreren zusätzlichen therapeutischen Mittel eine Verbindung umfassen ausgewählt aus:
(a) N-(5-Hydroxy-2,4-di-tert-butylphenyl)-4-oxo-1H-chinolin-3-carboxamid (Verbindung III): 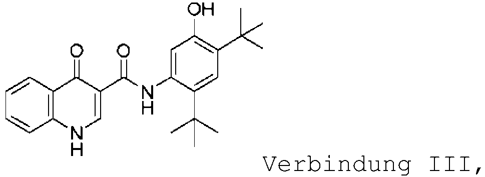
(b) N-(2-(tert-Butyl)-5-hydroxy-4-(2-(methyl-d3)propan-2-yl-1,1,1,3,3,3-d6)phenyl)-4-oxo-1,4-dihydrochinolin-3-carboxamid (Verbindung III-d): 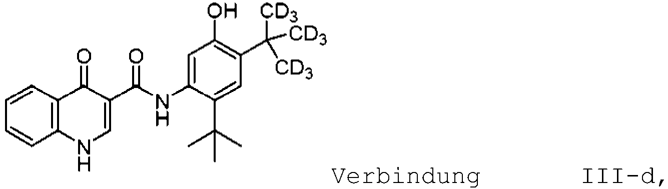 und deuterierte Derivate und pharmazeutisch annehmbare Salze davon.

11. Pharmazeutische Zusammensetzung, umfassend etwa 0,1 mg bis etwa 50 mg Verbindung I: 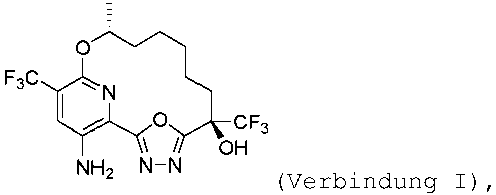 oder eine äquivalente Menge eines deuterierten Derivats oder eines pharmazeutisch annehmbaren Salzes davon.

12. Pharmazeutische Zusammensetzung nach Anspruch 11, wobei die Zusammensetzung umfasst:
etwa 0,25 mg bis etwa 50 mg Verbindung I oder eine äquivalente Menge eines deuterierten Derivats oder eines pharmazeutisch annehmbaren Salzes davon; beispielsweise etwa 0,25 mg bis etwa 25 mg Verbindung I oder eine äquivalente Menge eines deuterierten Derivats oder eines pharmazeutisch annehmbaren Salzes davon; beispielsweise etwa 1 mg bis etwa 15 mg Verbindung I oder eine äquivalente Menge eines deuterierten Derivats oder eines pharmazeutisch annehmbaren Salzes davon; beispielsweise etwa 1,5 mg bis etwa 14 mg Verbindung I oder eine äquivalente Menge eines deuterierten Derivats oder eines pharmazeutisch annehmbaren Salzes davon; beispielsweise etwa 4 mg bis etwa 13 mg Verbindung I oder eine äquivalente Menge eines deuterierten Derivats oder eines pharmazeutisch annehmbaren Salzes davon; beispielsweise etwa 8 mg bis etwa 13 mg Verbindung I oder eine äquivalente Menge eines deuterierten Derivats oder eines pharmazeutisch annehmbaren Salzes davon.

13. Pharmazeutische Zusammensetzung nach Anspruch 11, wobei die Zusammensetzung umfasst:
(a) etwa 10 mg bis etwa 40 mg Verbindung I oder eine äquivalente Menge eines deuterierten Derivats oder eines pharmazeutisch annehmbaren Salzes davon; oder
(b) etwa 0,1 mg, etwa 0,25 mg, etwa 1 mg, etwa 1,5 mg, etwa 2,5 mg, etwa 4 mg, etwa 4,5 mg, etwa 5 mg, etwa 5,5 mg, etwa 6 mg, etwa 6,5 mg, etwa 7 mg, etwa 7,5 mg, etwa 8 mg, etwa 8,5 mg, etwa 9, etwa 9,5 mg, etwa 10 mg, etwa 10,5 mg, etwa 11 mg, etwa 11,5 mg, etwa 12 mg, etwa 12,5 mg, etwa 13 mg, etwa 14 mg, etwa 15 mg, etwa 17,5 mg, etwa 20 mg, etwa 22,5 mg, etwa 25 mg, etwa 27,5 mg, etwa 30 mg, etwa 32,5 mg, etwa 35 mg, etwa 37,5 mg, etwa 40 mg, etwa 42,5 mg, etwa 45 mg, etwa 47,5 mg oder etwa 50 mg Verbindung I oder eine äquivalente Menge eines deuterierten Derivats oder eines pharmazeutisch annehmbaren Salzes davon.

14. Pharmazeutische Zusammensetzung nach einem der Ansprüche 11-13, wobei die Zusammensetzung ferner ein oder mehrere zusätzliche therapeutische Mittel umfasst.

15. Pharmazeutische Zusammensetzung nach Anspruch 14, wobei das eine oder die mehreren zusätzlichen therapeutischen Mittel eine Verbindung mit CFTRmodulierender Aktivität oder ein deuteriertes Derivat oder ein pharmazeutisch annehmbares Salz davon umfassen.

## Revendications

1. Composé I : 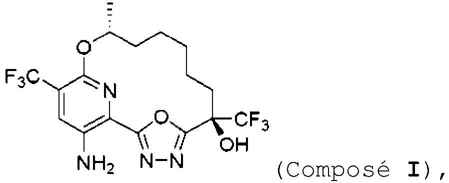 ou dérivé deutéré ou sel pharmaceutiquement acceptable de celui-ci, pour utilisation dans une méthode de traitement de la fibrose kystique chez un patient comprenant l'administration d'environ 0,1 mg à environ 50 mg de composé **I** ou d'une quantité équivalente d'un dérivé deutéré ou sel pharmaceutiquement acceptable de celui-ci par jour.

2. Composé pour utilisation selon la revendication 1, l'utilisation comprenant l'administration d'environ 0,25 mg à environ 50 mg de composé **I** ou d'une quantité équivalente d'un dérivé deutéré ou d'un sel pharmaceutiquement acceptable de celui-ci par jour ; par exemple,
d'environ 0,25 mg à environ 25 mg de composé **I** ou d'une quantité équivalente d'un dérivé deutéré ou d'un sel pharmaceutiquement acceptable de celui-ci par jour ; par exemple,
d'environ 1 mg à environ 15 mg de composé **I** ou d'une quantité équivalente d'un dérivé deutéré ou d'un sel pharmaceutiquement acceptable de celui-ci par jour ; par exemple,
d'environ 1,5 mg à environ 14 mg de composé **I** ou d'une quantité équivalente d'un dérivé deutéré ou d'un sel pharmaceutiquement acceptable de celui-ci par jour ; par exemple,
d'environ 4 mg à environ 13 mg de composé **I** ou d'une quantité équivalente d'un dérivé deutéré ou d'un sel pharmaceutiquement acceptable de celui-ci par jour ; par exemple,
d'environ 8 mg à environ 13 mg de composé **I** ou d'une quantité équivalente d'un dérivé deutéré ou d'un sel pharmaceutiquement acceptable de celui-ci par jour.

3. Composé pour utilisation selon la revendication 1 : (a) l'utilisation comprenant l'administration d'environ 10 mg à environ 40 mg de composé **I** ou d'une quantité équivalente d'un dérivé deutéré ou d'un sel pharmaceutiquement acceptable de celui-ci par jour ; ou (b) environ 0,1 mg, environ 0,25 mg, environ 1 mg, environ 1,5 mg, environ 2,5 mg, environ 4 mg, environ 4,5 mg, environ 5 mg, environ 5,5 mg, environ 6 mg, environ 6,5 mg, environ 7 mg, environ 7,5 mg, environ 8 mg, environ 8,5 mg, environ 9, environ 9,5 mg, environ 10 mg, environ 10,5 mg, environ 11 mg, environ 11,5 mg, environ 12 mg, environ 12,5 mg, environ 13 mg, environ 14 mg, environ 15 mg, environ 17,5 mg, environ 20 mg, environ 22,5 mg, environ 25 mg, environ 27,5 mg, environ 30 mg, environ 32,5 mg, environ 35 mg, environ 37,5 mg, environ 40 mg, environ 42,5 mg, environ 45 mg, environ 47,5 mg ou environ 50 mg de composé **I** ou une quantité équivalente d'un dérivé deutéré ou d'un sel pharmaceutiquement acceptable de celui-ci étant administrés par jour.

4. Composé pour utilisation selon la revendication 1, le patient étant hétérozygote et ayant une mutation F508del.

5. Composé pour utilisation selon la revendication 4, l'utilisation comprenant l'administration :
d'environ 0,25 mg à environ 50 mg de composé **I** ou d'une quantité équivalente d'un dérivé deutéré ou d'un sel pharmaceutiquement acceptable de celui-ci par jour ; par exemple,
d'environ 0,25 mg à environ 25 mg de composé **I** ou d'une quantité équivalente d'un dérivé deutéré ou d'un sel pharmaceutiquement acceptable de celui-ci par jour ; par exemple,
d'environ 1 mg à environ 15 mg de composé **I** ou d'une quantité équivalente d'un dérivé deutéré ou d'un sel pharmaceutiquement acceptable de celui-ci par jour ; par exemple,
d'environ 1,5 mg à environ 14 mg de composé **I** ou d'une quantité équivalente d'un dérivé deutéré ou d'un sel pharmaceutiquement acceptable de celui-ci par jour ; par exemple,
d'environ 4 mg à environ 13 mg de composé **I** ou d'une quantité équivalente d'un dérivé deutéré ou d'un sel pharmaceutiquement acceptable de celui-ci par jour ; par exemple,
d'environ 8 mg à environ 13 mg de composé **I** ou d'une quantité équivalente d'un dérivé deutéré ou d'un sel pharmaceutiquement acceptable de celui-ci par jour.

6. Composé pour utilisation selon la revendication 4 : (a) l'utilisation comprenant l'administration d'environ 10 mg à environ 40 mg de composé **I** ou d'une quantité équivalente d'un dérivé deutéré ou d'un sel pharmaceutiquement acceptable de celui-ci par jour ; ou (b) environ 0,1 mg, environ 0,25 mg, environ 1 mg, environ 1,5 mg, environ 2,5 mg, environ 4 mg, environ 4,5 mg, environ 5 mg, environ 5,5 mg, environ 6 mg, environ 6,5 mg, environ 7 mg, environ 7,5 mg, environ 8 mg, environ 8,5 mg, environ 9, environ 9,5 mg, environ 10 mg, environ 10,5 mg, environ 11 mg, environ 11,5 mg, environ 12 mg, environ 12,5 mg, environ 13 mg, environ 14 mg, environ 15 mg, environ 17,5 mg, environ 20 mg, environ 22,5 mg, environ 25 mg, environ 27,5 mg, environ 30 mg, environ 32,5 mg, environ 35 mg, environ 37,5 mg, environ 40 mg, environ 42,5 mg, environ 45 mg, environ 47,5 mg ou environ 50 mg de composé **I** ou une quantité équivalente d'un dérivé deutéré ou d'un sel pharmaceutiquement acceptable de celui-ci étant administrés par jour.

7. Composé pour utilisation selon l'une quelconque des revendications 1 à 6, le composé **I** ou un dérivé deutéré ou un sel pharmaceutiquement acceptable de celui-ci étant administrés :
(a) en une dose unique, une fois par jour ; et/ou
(b) en deux doses par jour.

8. Composé pour utilisation selon l'une quelconque des revendications 1 à 7, comprenant en outre l'administration d'un ou plusieurs agents thérapeutiques supplémentaires.

9. Composé pour utilisation selon la revendication 8, le ou les agents thérapeutiques supplémentaires comprenant un composé choisi parmi :
(a) le (*R*)-1-(2,2-difluorobenzo[d][1,3]dioxol-5-yl)-*N-*(1-(2,3-dihydroxypropyl)-6-fluoro-2-(1-hydroxy-2-méthylpropan-2-yl)-1*H*-indol-5-yl)cyclopropanecarboxamide (composé **II**) : 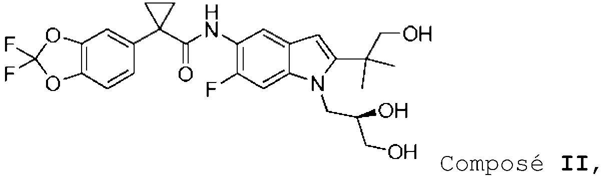
(b) l'acide 3-(6-(1-(2,2-difluorobenzo[d][1,3]dioxol-5-yl)cyclopropanecarboxamido)-3-méthylpyridin-2-yl)benzoïque (composé **IV**) : 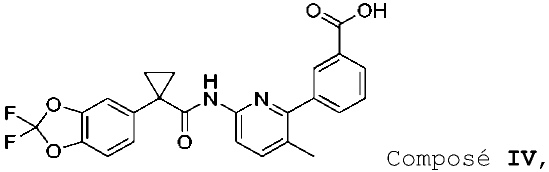
(c) le *N*-(1,3-diméthylpyrazol-4-yl)sulfonyl-6-[3-(3,3,3-trifluoro-2,2-diméthylpropoxy)pyrazol-1-yl]-2-[(4*S*)-2,2,4-triméthylpyrrolidin-1-yl]pyridine-3-carboxamide (composé **V**) : 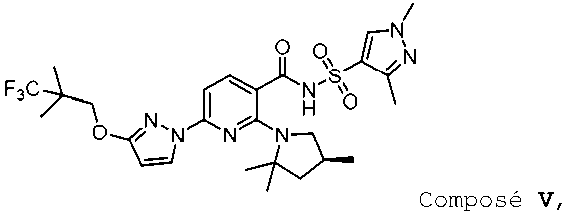
(d) le *N*-(benzènesulfonyl)-6-[3-[2-[1-(trifluorométhyl)cyclopropyl]éthoxy]pyrazol-1-yl]-2-[(4*S*)-2,2,4-triméthylpyrrolidin-1-yl]pyridine-3-carboxamide (composé VI) : 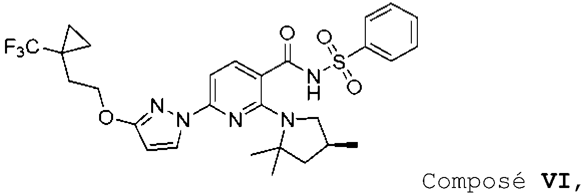
(e) la (14*S*)-8-[3-(2-{dispiro[2.0.2.1]heptan-7-yl}éthoxy)-1*H*-pyrazol-1-yl]-12,12-diméthyl-2λ⁶-thia-3,9,11,18,23-pentaazatétracyclo[17.3.1.111,14.05,10]tétracosa-1(22),5,7,9,19(23),20-hexaène-2,2,4-trione (composé **VII**) : 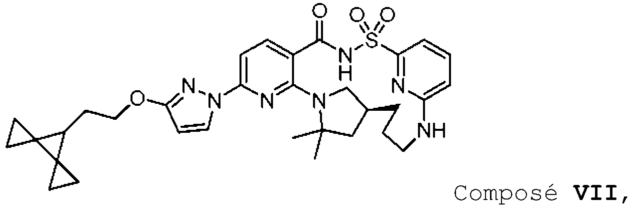
(f) la (11*R*)-6-(2,6-diméthylphényl)-11-(2-méthylpropyl)-12-{spiro[2.3]hexan-5-yl}-9-oxa-2λ⁶-thia-3,5,12,19-tétraazatricyclo[12.3.1.14,8]nonadéca-1(17),4(19),5,7,14(18),15-hexaène-2,2,13-trione (composé VIII) : 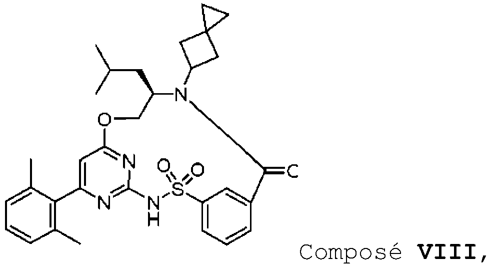
(g) le *N*-(benzènesulfonyl)-6-(3-fluoro-5-isobutoxyphényl)-2-[(4S)-2,2,4-triméthylpyrrolidin-1-yl]pyridine-3-carboxamide (composé **IX**) : 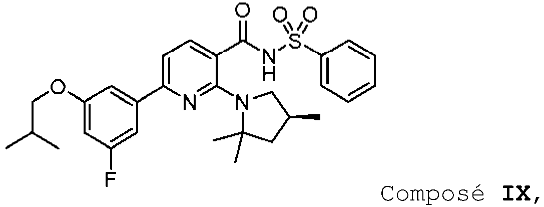
(h) le N-[(6-amino-2-pyridyl)sulfonyl]-6-(3-fluoro-5-isobutoxyphényl)-2-[(4*S*)-2,2,4-triméthylpyrrolidin-1-yl]pyridine-3-carboxamide (composé **X**) : 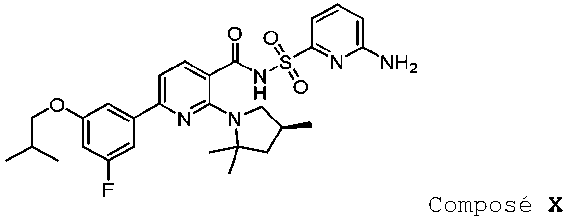 et les dérivés deutérés et sels pharmaceutiquement acceptables de ceuxci.

10. Composé pour utilisation selon la revendication 8 ou la revendication 9, le ou les agents thérapeutiques supplémentaires comprenant un composé choisi parmi :
(a) le *N*-(5-hydroxy-2,4-di-*tert*-butylphényl)-4-oxo-1H-quinoléine-3-carboxamide (composé **III**) : 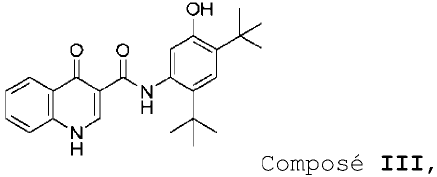
(b) le *N*-(2-(*tert*-butyl)-5-hydroxy-4-(2-(méthyl-d3)propan-2-yl-1,1,1,3,3,3-d6)phényl)-4-oxo-1,4-dihydroquinoléine-3-carboxamide (composé **III-d**) : 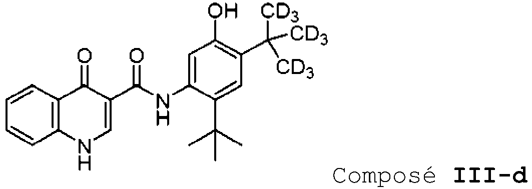 et les dérivés deutérés et sels pharmaceutiquement acceptables de ceuxci.

11. Composition pharmaceutique comprenant environ 0,1 mg à environ 50 mg de composé **I** : 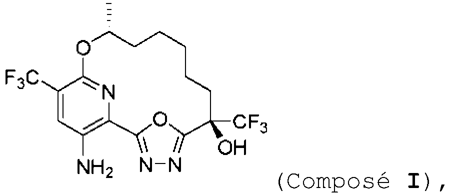 ou une quantité équivalente d'un dérivé deutéré ou d'un sel pharmaceutiquement acceptable de celui-ci.

12. Composition pharmaceutique selon la revendication 11, la composition comprenant :
environ 0,25 mg à environ 50 mg de composé **I** ou une quantité équivalente d'un dérivé deutéré ou d'un sel pharmaceutiquement acceptable de celui-ci ; par exemple, environ 0,25 mg à environ 25 mg de composé **I** ou une quantité équivalente d'un dérivé deutéré ou d'un sel pharmaceutiquement acceptable de celui-ci ; par exemple, environ 1 mg à environ 15 mg de composé **I** ou une quantité équivalente d'un dérivé deutéré ou d'un sel pharmaceutiquement acceptable de celui-ci ; par exemple, environ 1,5 mg à environ 14 mg de composé **I** ou une quantité équivalente d'un dérivé deutéré ou d'un sel pharmaceutiquement acceptable de celui-ci ; par exemple, environ 4 mg à environ 13 mg de composé **I** ou une quantité équivalente d'un dérivé deutéré ou d'un sel pharmaceutiquement acceptable de celui-ci ; par exemple, environ 8 mg à environ 13 mg de composé **I** ou une quantité équivalente d'un dérivé deutéré ou d'un sel pharmaceutiquement acceptable de celui-ci.

13. Composition pharmaceutique selon la revendication 11, la composition comprenant :
(a) environ 10 mg à environ 40 mg de composé **I** ou une quantité équivalente d'un dérivé deutéré ou d'un sel pharmaceutiquement acceptable de celui-ci ; ou
(b) environ 0,1 mg, environ 0,25 mg, environ 1 mg, environ 1,5 mg, environ 2,5 mg, environ 4 mg, environ 4,5 mg, environ 5 mg, environ 5,5 mg, environ 6 mg, environ 6,5 mg, environ 7 mg, environ 7,5 mg, environ 8 mg, environ 8,5 mg, environ 9, environ 9,5 mg, environ 10 mg, environ 10,5 mg, environ 11 mg, environ 11,5 mg, environ 12 mg, environ 12,5 mg, environ 13 mg, environ 14 mg, environ 15 mg, environ 17,5 mg, environ 20 mg, environ 22,5 mg, environ 25 mg, environ 27,5 mg, environ 30 mg, environ 32,5 mg, environ 35 mg, environ 37,5 mg, environ 40 mg, environ 42,5 mg, environ 45 mg, environ 47,5 mg ou environ 50 mg de composé **I** ou une quantité équivalente d'un dérivé deutéré ou d'un sel pharmaceutiquement acceptable de celui-ci.

14. Composition pharmaceutique selon l'une quelconque des revendications 11 à 13, la composition comprenant en outre un ou plusieurs agents thérapeutiques supplémentaires.

15. Composition pharmaceutique selon la revendication 14, le ou les agents thérapeutiques supplémentaires comprenant un composé ayant une activité de modulation de CFTR ou un dérivé deutéré ou un sel pharmaceutiquement acceptable de celui-ci.
